(19) Europäisches Patentamt
European Patent Office
Office européen des brevets

(11) EP 1 581 574 B1

(12) # EUROPEAN PATENT SPECIFICATION

(45) Date of publication and mention of the grant of the patent:
**12.04.2017 Bulletin 2017/15**

(21) Application number: **03814645.2**

(22) Date of filing: **03.12.2003**

(51) Int Cl.:
***C08G 18/12*** *(2006.01)* ***C08G 18/10*** *(2006.01)*
***G02B 1/04*** *(2006.01)*

(86) International application number:
**PCT/US2003/038436**

(87) International publication number:
**WO 2004/060951 (22.07.2004 Gazette 2004/30)**

(54) **HIGH IMPACT POLY(URETHANE UREA) POLYSULFIDES**

SCHLAGZÄHE POLY(URETHANHARNSTOFF)POLYSULFIDE

POLYSULFURES DE CARBAMIDE DE (POLY)URETHANE) TRES RESISTANTS

(84) Designated Contracting States:
**AT BE BG CH CY CZ DE DK EE ES FI FR GB GR HU IE IT LI LU MC NL PT RO SE SI SK TR**

(30) Priority: **20.12.2002 US 435537 P**
**02.12.2003 US 725023**

(43) Date of publication of application:
**05.10.2005 Bulletin 2005/40**

(60) Divisional application:
**11001940.3 / 2 325 233**
**11001941.1 / 2 325 234**

(73) Proprietor: **PPG Industries Ohio, Inc.**
**Cleveland, OH 44111 (US)**

(72) Inventors:
- **BOJKOVA, Nina**
  **Monroeville, PA 15146 (US)**
- **SMITH, Robert, A.**
  **Murrysville, PA 15668 (US)**
- **HEROLD, Robert, D.**
  **Monroeville, PA 15146 (US)**
- **RAO, Chandra**
  **Valencia, CA 91355 (US)**
- **McDONALD, William, H.**
  **Mars, Pennsylvania 16046 (US)**
- **NAGPAL, Vidhu, J.**
  **Murrysville, PA 15668 (US)**
- **GRAHAM, Marvin, J.**
  **Monroeville, PA 15146 (US)**
- **YU, Phillip, C.**
  **Pittsburgh, PA 15239 (US)**
- **SAWANT, Suresh**
  **Stevenson Ranch, CA 91381 (US)**
- **OKOROAFOR, Michael, O.**
  **Roswell, GA 30075 (US)**

(74) Representative: **f & e patent**
**Fleischer, Engels & Partner mbB, Patentanwälte**
**Braunsberger Feld 29**
**51429 Bergisch Gladbach (DE)**

(56) References cited:
**EP-A- 1 384 736** **WO-A-00/14137**
**WO-A-01/36507** **WO-A-01/36508**
**WO-A-03/042270** **WO-A-03/044070**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to sulfur-containing polyureaurethanes and methods for their preparation.

A number of organic polymeric materials, such as plastics, have been developed as alternatives and replacements for glass in applications such as optical lenses, fiber optics, windows and automotive, nautical and aviation transparencies. These polymeric materials can provide advantages relative to glass, including, shatter resistance, lighter weight for a given application, ease of molding and ease of dying. However, the refractive indices of many polymeric materials are generally lower than that of glass. In ophthalmic applications, the use of a polymeric material having a lower refractive index will require a thicker lens relative to a material having a higher refractive index. A thicker lens is not desirable.

Thus, there is a need in the art to develop a polymeric material having an adequate refractive index and good impact resistance/strength.

The present invention is directed to a sulfur-containing polyureaurethane as defined by claim 1.

At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.

As used herein and the claims, the term "cyanate" refers to isocyanate materials and isothiocyanate materials that are unblocked and capable of forming a covalent bond with a reactive group such as a thiol, hydroxyl, or amine function group. In a non-limiting embodiment, the polycyanate of the present invention can contain at least two functional groups chosen from isocyanate (NCO), isothiocyanate (NCS), and combinations of isocyanate and isothiocyanate functional groups. The term "isocyanate" refers to a cyanate which is free of sulfur. The term "isothiocyanate" refers to a sulfur-containing cyanate.

In alternate non-limiting embodiments, the polyureaurethane of the invention when polymerized can produce a polymerizate having a refractive index of at least 1.57, or at least 1.58, or at least 1.60, or at least 1.62. In further alternate non-limiting embodiments, the polyureaurethane of the invention when polymerized can produce a polymerizate having an Abbe number of at least 32, or at least 35, or at least 38, or at least 39, or at least 40, or at least 44. The refractive index and Abbe number can be determined by methods known in the art such as American Standard Test Method (ASTM) Number D 542-00. Further, the refractive index and Abbe number can be determined using various known instruments. In a non-limiting embodiment of the present invention, the refractive index and Abbe number can be measured in accordance with ASTM D 542-00 with the following exceptions: (i) test one to two samples/specimens instead of the minimum of three specimens specified in Section 7.3; and (ii) test the samples unconditioned instead of conditioning the samples/specimens prior to testing as specified in Section 8.1. Further, in a non-limiting embodiment, an Atago, model DR-M2 Multi-Wavelength Digital Abbe Refractometer can be used to measure the refractive index and Abbe number of the samples/specimens.

In alternate non-limiting embodiments, the amount of polycyanate and the amount of active hydrogen-containing material can be selected such that the equivalent ratio of (NCO + NCS):(SH + OH) can be greater than 1.0:1.0, or at least 2.0:1.0, or at least 2.5:1, or less than 4.5:1.0, or less than 5.5:1.0. In further alternate non-limiting embodiments, the equivalent ratio of (NCO + NCS)(SH + OH + NR), wherein R can be hydrogen or alkyl, can be greater than 1.0:1.0, or at least 2.0:1.0, or at least 2.5:1, or less than 4.5:1.0, or less than 5.5:1.0.

Polycyanates useful in the preparation of the polyureaurethane of the present invention are numerous and widely varied. Suitable polycyanates for use in the present invention can include but are not limited to polymeric and $C_2$-$C_{20}$ linear, branched, cyclic and aromatic polycyanates. Non-limiting examples can include polyisocyanates and polyisothiocyanates having backbone linkages chosen from urethane linkages (-NH-C(O)-O-), thiourethane linkages (-NH-C(O)-S-), thiocarbamate linkages (-NH-C(S)-O-), dithiourethane linkages (-NH-C(S)-S-) and combinations thereof.

The molecular weight of the polycyanate can vary widely. In alternate non-limiting embodiments, the number average molecular weight (Mn) can be at least 100 grams/mole, or at least 150 grams/mole, or less than 15,000 grams/mole, or less than 5000 grams/mole. The number average molecular weight can be determined using known methods. The number average molecular weight values recited herein and the claims were determined by gel permeation chromatography (GPC) using polystyrene standards.

Non-limiting examples of suitable polycyanates can include but are not limited to polyisocyanates having at least two isocyanate groups; isothiocyanates having at least two isothiocyanate groups; mixtures thereof; and combinations thereof, such as a material having isocyanate and isothiocyanate functionality.

Non-limiting examples of polyisocyanates can include but are not limited to aliphatic polyisocyanates, cycloaliphatic polyisocyanates wherein one or more of the isocyanato groups are attached directly to the cycloaliphatic

ring, cycloaliphatic polyisocyanates wherein one or more of the isocyanato groups are not attached directly to the cycloaliphatic ring, aromatic polyisocyanates wherein one or more of the isocyanato groups are attached directly to the aromatic ring, and aromatic polyisocyanates wherein one or more of the isocyanato groups are not attached directly to the aromatic ring. When an aromatic polyisocyanate is used, generally care should be taken to select a material that does not cause the polyureaurethane to color (e.g., yellow).

In a non-limiting embodiment of the present invention, the polyisocyanate can include but is not limited to aliphatic or cycloaliphatic diisocyanates, aromatic diisocyanates, cyclic dimmers and cyclic trimers thereof, and mixtures thereof. Non-limiting examples of suitable polyisocyanates can include but are not limited to Desmodur N 3300 (hexamethylene diisocyanate trimer) which is commercially available from Bayer; Desmodur N 3400 (60% hexamethylene diisocyanate dimer and 40% hexamethylene diisocyanate trimer).

In a non-limiting embodiment, the polyisocyanate can include dicyclohexylmethane diisocyanate and isomeric mixtures thereof. As used herein and the claims, the term "isomeric mixtures" refers to a mixture of the cis-cis, trans-trans, and cis-trans isomers of the polyisocyanate. Non-limiting examples of isomeric mixtures for use in the present invention can include the trans-trans isomer of 4,4'-methylenebis(cyclohexyl isocyanate), hereinafter referred to as "PICM" (paraisocyanato cyclohexylmethane), the cis-trans isomer of PICM, the cis-cis isomer of PICM, and mixtures thereof.

In one non-limiting embodiment, three suitable isomers of 4,4'-methylenebis(cyclohexyl isocyanate) for use in the present invention are shown below.

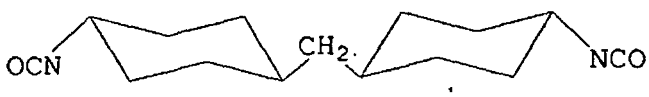

trans, trans

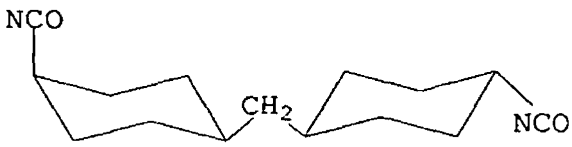

cis, trans

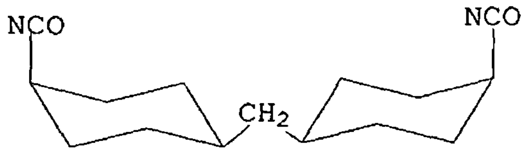

cis, cis

In one non-limiting embodiment, the PICM used in this invention can be prepared by phosgenating the 4,4'-methylenebis(cyclohexyl amine) (PACM) by procedures well known in the art such as the procedures disclosed in United States Patents 2,644,007 and 2,680,127. The PACM isomer mixtures, upon phosgenation, can produce PICM in a liquid phase, a partially liquid phase, or a solid phase at room temperature. The PACM isomer mixtures can be obtained by the hydrogenation of methylenedianiline and/or by fractional crystallization of PACM isomer mixtures in the presence of water and alcohols such as methanol and ethanol.

In a non-limiting embodiment, the isomeric mixture can contain from 10-100 percent of the trans,trans isomer of 4,4'-methylenebis(cyclohexyl isocyanate) (PICM).

Additional aliphatic and cycloaliphatic diisocyanates that can be used in alternate non-limiting embodiments of the present invention include 3-isocyanatomethyl-3,5,5-trimethyl cyclohexyl-isocyanate ("IPDI") which is commercially available from Arco Chemical, and meta-tetramethylxylene diisocyanate (1,3-bis(1-isocyanato-1-methylethyl)-benzene) which is commercially available from Cytec Industries Inc. under the tradename TMXDI.RTM. (Meta) Aliphatic Isocyanate.

As used herein and the claims, the terms aliphatic and cycloaliphatic diisocyanates refer to 6 to 100 carbon atoms linked in a straight chain or cyclized having two diisocyanate reactive end groups. In a non-limiting embodiment of the present invention, the aliphatic and cycloaliphatic diisocyanates for use in the present invention can include TMXDI and compounds of the formula $R\text{-}(NCO)_2$ wherein R represents an aliphatic group or a cycloaliphatic group.

Further non-limiting examples of suitable polycyanates can include but are not limited to aliphatic polyisocyanates and polyisothiocyanates; ethylenically unsaturated polyisocyanates and polyisothiocyanates; alicyclic polyisocyanates and polyisothiocyanates; aromatic polyisocyanates and polyisothiocyanates wherein the isocyanate groups are not bonded directly to the aromatic ring, e.g., α,α'-xylene diisocyanate; aromatic polyisocyanates and polyisothiocyanates wherein the isocyanate groups are bonded directly to the aromatic ring, e.g., benzene diisocyanate; aliphatic polyisocyanates and polyisothiocyanates containing sulfide linkages; aromatic polyisocyanates and polyisothiocyanates containing sulfide or disulfide linkages; aromatic polyisocyanates and polyisothiocyanates containing sulfone linkages; sulfonic ester-type polyisocyanates and polyisothiocyanates, e.g., 4-methyl-3-isocyanatobenzenesulfonyl-4'-isocyanatophenol ester; aromatic sulfonic amide-type polyisocyanates and polyisothiocyanates; sulfur-containing heterocyclic polyisocyanates and polyisothiocyanates, e.g., thiophene-2,5-diisocyanate; halogenated, alkylated, alkoxylated, nitrated, carbodiimide modified, urea modified and biuret modified derivatives of polycyanates thereof; and dimerized and trimerized products of polycyanates thereof.

In a further non-limiting embodiment, a material of the following general formula (I) can be used in preparation of the polyurethane prepolymer:

(I) 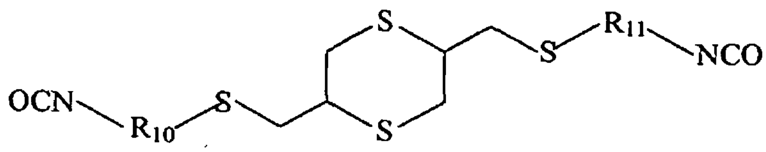

wherein $R_{10}$ and $R_{11}$ are each independently $C_1$ to $C_3$ alkyl.

Further non-limiting examples of aliphatic polyisocyanates can include ethylene diisocyanate, trimethylene diisocyanate, tetramethylene diisocyanate, hexamethylene diisocyanate, octamethylene diisocyanate, nonamethylene diisocyanate, 2,2'-dimethylpentane diisocyanate, 2,2,4-trimethylhexane diisocyanate, decamethylene diisocyanate, 2,4,4,-trimethylhexamethylene diisocyanate, 1,6,11-undecanetriisocyanate, 1,3,6-hexamethylene triisocyanate, 1,8-diisocyanato-4-(isocyanatomethyl)octane, 2,5,7-trimethyl-1,8-diisocyanato-5-(isocyanatomethyl)octane, bis(isocyanatoethyl)-carbonate, bis(isocyanatoethyl)ether, 2-isocyanatopropyl-2,6-diisocyanatohexanoate, lysinediisocyanate methyl ester and lysinetriisocyanate methyl ester.

Examples of ethylenically unsaturated polyisocyanates can include but are not limited to butene diisocyanate and 1,3-butadiene-1,4-diisocyanate. Alicyclic polyisocyanates can include but are not limited to isophorone diisocyanate, cyclohexane diisocyanate, methylcyclohexane diisocyanate, bis(isocyanatomethyl) cyclohexane, bis(isocyanatocyclohexyl)methane, bis(isocyanatocyclohexyl)-2,2-propane, bis(isocyanatocyclohexyl)-1,2-ethane, 2-isocyanatomethyl-3-(3-isocyanatopropyl)-5-isocyanatomethyl-bicyclo[2.2.1]-heptane, 2-isocyanatomethyl-3-(3-isocyanatopropyl)-6-isocyanatomethyl-bicyclo[2.2.1]-heptane, 2-isocyanatomethyl-2-(3-isocyanatopropyl)-5-isocyanatomethyl-bicyclo[2.2.1]-heptane, 2-isocyanatomethyl-2-(3-isocyanatopropyl)-6-isocyanatomethyl-bicyclo[2.2.1]-heptane, 2-isocyanatomethyl-3-(3-isocyanatopropyl)-6-(2-isocyanatoethyl)-bicyclo[2.2.1]-heptane, 2-isocyanatomethyl-2-(3-isocyanatopropyl)-5-(2-isocyanatoethyl)-bicyclo[2.2.1]-heptane and 2-isocyanatomethyl-2-(3-isocyanatopropyl)-6-(2-isocyanatoethyl)-bicyclo[2.2.1]-heptane.

Examples of aromatic polyisocyanates wherein the isocyanate groups are not bonded directly to the aromatic ring can include but are not limited to bis(isocyanatoethyl)benzene, α,α,α',α'-tetramethylxylene diisocyanate, 1,3-bis(1-isocyanato-1-methylethyl)benzene, bis(isocyanatobutyl)benzene, bis(isocyanatomethyl)naphthalene, bis(isocyanatomethyl)diphenyl ether, bis(isocyanatoethyl) phthalate, mesitylene triisocyanate and 2,5-di(isocyanatomethyl)furan. Aromatic polyisocyanates having isocyanate groups bonded directly to the aromatic ring can include but are not limited to phenylene diisocyanate, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, naphthalene diisocyanate, methylnaphthalene diisocyanate, biphenyl diisocyanate, ortho- toluidine diisocyanate, ortho-tolylidine diisocyanate, ortho-tolylene diisocyanate, 4,4'-diphenylmethane diisocyanate, bis(3-methyl-4-isocyanatophenyl)methane, bis(isocyanatophenyl)ethylene, 3,3'-dimethoxy-biphenyl-4,4'-diisocyanate, triphenylmethane triisocyanate, polymeric 4,4'-diphenylmethane diisocyanate, naphthalene triisocyanate, diphenylmethane-2,4,4'-triisocyanate, 4-methyldiphenylmethane-3,5,2',4',6'-pentaisocyanate, diphenylether diisocyanate, bis(isocyanatophenylether)ethyleneglycol, bis(isocyanatophenylether)-1,3-propyleneglycol, benzophenone diisocyanate, carbazole diisocyanate, ethylcarbazole diisocyanate and dichlorocarbazole diisocyanate.

Further non-limiting examples of aliphatic and cycloaliphatic diisocyanates that can be used in the present invention include 3-isocyanato-methyl-3,5,5-trimethyl cyclohexyl-isocyanate ("IPDI") which is commercially available from Arco Chemical, and meta-tetramethylxylene diisocyanate (1,3-bis(1-isocyanato-1-methylethyl)-benzene) which is commercially available from Cytec Industries Inc. under the tradename TMXDI.RTM. (Meta) Aliphatic Isocyanate.

In a non-limiting embodiment of the present invention, the aliphatic and cycloaliphatic diisocyanates for use in the present invention can include TMXDI and compounds of the formula R-$(NCO)_2$ wherein R represents an aliphatic

group or a cycloaliphatic group.

Non-limiting examples of polyisocyanates can include aliphatic polyisocyanates containing sulfide linkages such as thiodiethyl diisocyanate, thiodipropyl diisocyanate, dithiodihexyl diisocyanate, dimethylsulfone diisocyanate, dithiodimethyl diisocyanate, dithiodiethyl diisocyanate, dithiodipropyl diisocyanate and dicyclohexylsulfide-4,4'-diisocyanate. Non-limiting examples of aromatic polyisocyanates containing sulfide or disulfide linkages include but are not limited to diphenylsulfide-2,4'-diisocyanate, diphenylsulfide-4,4'-diisocyanate, 3,3'-dimethoxy-4,4'-diisocyanatodibenzyl thioether, bis(4-isocyanatomethylbenzene)-sulfide, diphenyldisulfide-4,4'-diisocyanate, 2,2'-dimethyldiphenyldisulfide-5,5'-diisocyanate, 3,3'-dimethyldiphenyldisulfide-5,5'-diisocyanate, 3,3'-dimethyldiphenyldisulfide-6,6'-diisocyanate, 4,4'-dimethyldiphenyldisulfide-5,5'-diisocyanate, 3,3'-dimethoxydiphenyldisulfide-4,4'-diisocyanate and 4,4'-dimethoxydiphenyldisulfide-3,3'-diisocyanate.

Non-limiting examples polyisocyanates can include aromatic polyisocyanates containing sulfone linkages such as diphenylsulfone-4,4'-diisocyanate, diphenylsulfone-3,3'-diisocyanate, benzidinesulfone-4,4'-diisocyanate, diphenylmethanesulfone-4,4'-diisocyanate, 4-methyldiphenylmethanesulfone-2,4'-diisocyanate, 4,4'-dimethoxydiphenylsulfone-3,3'-diisocyanate, 3,3'-dimethoxy-4,4'-diisocyanatodibenzylsulfone, 4,4'-dimethyldiphenylsulfone-3,3'-diisocyanate, 4,4'-di-tert-butyl-diphenylsulfone-3,3'-diisocyanate and 4,4'-dichlorodiphenylsulfone-3,3'-diisocyanate.

Non-limiting examples of aromatic sulfonic amide-type polyisocyanates for use in the present invention can include 4-methyl-3-isocyanato-benzene-sulfonylanilide-3'-methyl-4'-isocyanate, dibenzenesulfonyl-ethylenediamine-4,4'-diisocyanate, 4,4'-methoxybenzenesulfonyl-ethylenediamine-3,3'-diisocyanate and 4-methyl-3-isocyanato-benzene-sulfonylanilide-4-ethyl-3'-isocyanate.

In alternate non-limiting embodiments, the polyisothiocyanate can include aliphatic polyisothiocyanates; alicyclic polyisothiocyanates, such as but not limited to cyclohexane diisothiocyanates; aromatic polyisothiocyanates wherein the isothiocyanate groups are not bonded directly to the aromatic ring, such as but not limited to $\alpha,\alpha'$-xylene diisothiocyanate; aromatic polyisothiocyanates wherein the isothiocyanate groups are bonded directly to the aromatic ring, such as but not limited to phenylene diisothiocyanate; heterocyclic polyisothiocyanates, such as but not limited to 2,4,6-triisothicyanato-1,3,5-triazine and thiophene-2,5-diisothiocyanate; carbonyl polyisothiocyanates; aliphatic polyisothiocyanates containing sulfide linkages, such as but not limited to thiobis(3-isothiocyanatopropane); aromatic polyisothiocyanates containing sulfur atoms in addition to those of the isothiocyanate groups; halogenated, alkylated, alkoxylated, nitrated, carbodiimide modified, urea modified and biuret modified derivatives of these polyisothiocyanates; and dimerized and trimerized products of these polyisothiocyanates.

Non-limiting examples of aliphatic polyisothiocyanates include 1,2-diisothiocyanatoethane, 1,3-diisothiocyanatopropane, 1,4-diisothiocyanatobutane and 1,6-diisothiocyanatohexane. Non-limiting examples of aromatic polyisothiocyanates having isothiocyanate groups bonded directly to the aromatic ring can include but are not limited to 1,2-diisothiocyanatobenzene, 1,3-diisothiocyanatobenzene, 1,4-diisothiocyanatobenzene, 2,4-diisothiocyanatotoluene, 2,5-diisothiocyanato-m-xylene, 4,4'-diisothiocyanato-1,1'-biphenyl, 1,1'-methylenebis(4-isothiocyanatobenzene), 1,1'-methylenebis(4-isothiocyanato-2-methylbenzene), 1,1'-methylenebis(4-isothiocyanato-3-methylbenzene), 1,1'-(1,2-ethane-diyl)bis(4-isothiocyanatobenzene), 4,4'-diisothiocyanatobenzophenenone, 4,4'-diisothiocyanato-3,3'-dimethylbenzophenone, benzanilide-3,4'-diisothiocyanate, diphenylether-4,4'-diisothiocyanate and diphenylamine-4,4'-diisothiocyanate.

Suitable carbonyl polyisothiocyanates can include but are not limited to hexane-dioyl diisothiocyanate, nonanedioyl diisothiocyanate, carbonic diisothiocyanate, 1,3-benzenedicarbonyl diisothiocyanate, 1,4-benzenedicarbonyl diisothiocyanate and (2,2'-bipyridine)-4,4'-dicarbonyl diisothiocyanate. Non-limiting examples of aromatic polyisothiocyanates containing sulfur atoms in addition to those of the isothiocyanate groups, can include but are not limited to 1-isothiocyanato-4-[(2-isothiocyanato)sulfonyl]benzene, thiobis(4-isothiocyanatobenzene), sulfonylbis(4-isothiocyanatobenzene), sulfinylbis(4-isothiocyanatobenzene), dithiobis(4-isothiocyanatobenzene), 4-isothiocyanato-1-[(4-isothiocyanatophenyl)-sulfonyl]-2-methoxybenzene, 4-methyl-3-isothicyanatobenzene-sulfonyl-4'-isothiocyanate phenyl ester and 4-methyl-3-isothiocyanatobenzene-sulfonylanilide-3'-methyl-4'-isothiocyanate.

Non-limiting examples of polycyanates having isocyanate and isothiocyanate groups can include aliphatic, alicyclic, aromatic, heterocyclic, or contain sulfur atoms in addition to those of the isothiocyanate groups. Non-limiting examples of such polycyanates include but are not limited to 1-isocyanato-3-isothiocyanatopropane, 1-isocyanato-5-isothiocyanatopentane, 1-isocyanato-6-isothiocyanatohexane, isocyanatocarbonyl isothiocyanate, 1-isocyanato-4-isothiocyanatocyclohexane, 1-isocyanato-4-isothiocyanatobenzene, 4-methyl-3-isocyanato-1-isothiocyanatobenzene, 2-isocyanato-4,6-diisothiocyanato-1,3,5-triazine, 4-isocyanato-4'-isothiocyanato-diphenyl sulfide and 2-isocyanato-2'-isothiocyanatodiethyl disulfide.

In a non-limiting embodiment, the polycyanate can be reacted with an active hydrogen-containing material to form a polyurethane prepolymer. Active hydrogen-containing materials are varied and known in the art. Non-limiting examples can include hydroxyl-containing materials such as but not limited to polyols; sulfur-containing materials such as but not limited to hydroxyl functional polysulfides, and SH-containing materials such as but not limited to polythiols; and materials having both hydroxyl and thiol functional groups.

Suitable hydroxyl-containing materials for use in the present invention can include a wide variety of materials known in the art. Non-limiting examples can include but are not limited to polyether polyols, polyester polyols, polycaprolactone polyols, polycarbonate polyols, and mixtures thereof.

Polyether polyols and methods for their preparation are known to one skilled in the art. Many polyether polyols of various types and molecular weight are commercially available from various manufacturers. Non-limiting examples of polyether polyols can include but are not limited to polyoxyalkylene polyols, and polyalkoxylated polyols. Polyoxyalkylene polyols can be prepared in accordance with known methods. In a non-limiting embodiment, a polyoxyalkylene polyol can be prepared by condensing an alkylene oxide, or a mixture of alkylene oxides, using acid- or base-catalyzed addition with a polyhydric initiator or a mixture of polyhydric initiators, such as but not limited to ethylene glycol, propylene glycol, glycerol, and sorbitol. Non-limiting examples of alkylene oxides can include ethylene oxide, propylene oxide, butylene oxide, amylene oxide, aralkylene oxides, such as but not limited to styrene oxide, mixtures of ethylene oxide and propylene oxide. In a further non-limiting embodiment, polyoxyalkylene polyols can be prepared with mixtures of alkylene oxide using random or step-wise oxyalkylation. Non-limiting examples of such polyoxyalkylene polyols include polyoxyethylene, such as but not limited to polyethylene glycol, polyoxypropylene, such as but not limited to polypropylene glycol.

In a non-limiting embodiment, polyalkoxylated polyols can be represent by the following general formula:

$$(I') \quad H-(O-\underset{\substack{|\\R_1}}{CH}-CH_2)_m-O-A-O-(CH_2-\underset{\substack{|\\R_2}}{CH}-)_n-OH$$

wherein m and n can each be a positive integer, the sum of m and n being from 5 to 70; $R_1$ and $R_2$ are each hydrogen, methyl or ethyl; and A is a divalent linking group such as a straight or branched chain alkylene which can contain from 1 to 8 carbon atoms, phenylene, and $C_1$ to $C_9$ alkyl-substituted phenylene. The chosen values of m and n can, in combination with the chosen divalent linking group, determine the molecular weight of the polyol. Polyalkoxylated polyols can be prepared by methods that are known in the art. In a non-limiting embodiment, a polyol such as 4,4'-isopropylidenediphenol can be reacted with an oxirane-containing material such as but not limited to ethylene oxide, propylene oxide and butylene oxide, to form what is commonly referred to as an ethoxylated, propoxylated or butoxylated polyol having hydroxy functionality. Non-limiting examples of polyols suitable for use in preparing polyalkoxylate polyols can include those polyols described in United States Patent 6,187,444 B1 at column 10, lines 1-20.

As used herein and the claims, the term "polyether polyols" can include the generally known poly(oxytetramethylene) diols prepared by the polymerization of tetrahydrofuran in the presence of Lewis acid catalysts such as but not limited to boron trifluoride, tin (IV) chloride and sulfonyl chloride. Also included are the polyethers prepared by the copolymerization of cyclic ethers such as but not limited to ethylene oxide, propylene oxide, trimethylene oxide, and tetrahydrofuran with aliphatic diols such as but not limited to ethylene glycol, 1,3-butanediol, 1,4-butanediol, diethylene glycol, dipropylene glycol, 1,2-propylene glycol and 1,3-propylene glycol. Compatible mixtures of polyether polyols can also be used. As used herein, "compatible" means that the polyols are mutually soluble in each other so as to form a single phase.

A variety of polyester polyols for use in the present invention are known in the art. Suitable polyester polyols can include but are not limited to polyester glycols. Polyester glycols for use in the present invention can include the esterification products of one or more dicarboxylic acids having from four to ten carbon atoms, such as but not limited to adipic, succinic or sebacic acids, with one or more low molecular weight glycols having from two to ten carbon atoms, such as but not limited to ethylene glycol, propylene glycol, diethylene glycol, 1,4-butanediol, neopentyl glycol, 1,6-hexanediol and 1,10-decanediol. Esterification procedures for producing polyester polyols is described, for example, in the article D.M. Young, F. Hostettler et al., "Polyesters from Lactone," Union Carbide F-40, p. 147.

In a non-limiting embodiment, the polyol for use in the present invention can include polycaprolactone polyols. Suitable polycaprolactone polyols are varied and known in the art. In a non-limiting embodiment, polycaprolactone polyols can be prepared by condensing caprolactone in the presence of difunctional active hydrogen compounds such as but not limited to water or low molecular weight glycols as recited herein. Non-limiting examples of suitable polycaprolactone polyols can include commercially available materials designated as the CAPA series from Solvay Chemical which includes but is not limited to CAPA 2047A, and the TONE series from Dow Chemical such as but not limited to TONE 0201.

Polycarbonate polyols for use in the present invention are varied and known to one skilled in the art. Suitable polycarbonate polyols can include those commercially available (such as but not limited to Ravecarb™ 107 from Enichem S.p.A.). In a non-limiting embodiment, the polycarbonate polyol can be produced by reacting an organic glycol such as a diol, described hereinafter and in connection with the glycol component of the polyureaurethane, and a dialkyl carbonate, such as described in United States Patent 4,160,853. In a non-limiting embodiment, the polyol can include polyhexamethyl

carbonate such as $HO-(CH_2)_6-[O-C(O)-O-(CH_2)_6]_n-OH$, wherein n is an integer from 4 to 24, or from 4 to 10, or from 5 to 7.

In a non-limiting embodiment, the glycol material can comprise low molecular weight polyols such as polyols having a number average molecular weight of less than 500 grams/mole, and compatible mixtures thereof. As used herein, "compatible" means that the glycols are mutually soluble in each other so as to form a single phase. Non-limiting examples of these polyols can include but are not limited to low molecular weight diols and triols. In a further non-limiting embodiment, the amount of triol chosen is such to avoid a high degree of cross-linking in the polyurethane. The organic glycol typically contains from 2 to 16, or from 2 to 6, or from 2 to 10, carbon atoms. Non-limiting examples of such glycols can include but are not limited to ethylene glycol, propylene glycol, diethylene glycol, triethylene glycol, tetraethylene glycol, dipropylene glycol, tripropylene glycol, 1,2-, 1,3- and 1,4-butanediol, 2,2,4-trimethyl-1,3-pentanediol, 2-methyl-1,3-pentanediol, 1,3-2,4- and 1,5-pentanediol, 2,5- and 1,6-hexanediol, 2,4-heptanediol, 2-ethyl-1,3-hexanediol, 2,2-dimethyl-1,3-propanediol, 1,8-octanediol, 1,9-nonanediol, 1,10-decanediol, 1,4-cyclohexanediol, 1,4-cyclohexanedimethanol, 1,2-bis(hydroxyethyl)-cyclohexane, glycerin, tetramethylolmethane, such as but not limited to pentaerythritol, trimethylolethane and trimethylolpropane; and isomers thereof.

In alternate non-limiting embodiments, the hydroxyl-containing material can have a molecular weight of at least 200 grams/mole, or at least 1000 grams/mole, or at least 2000 grams/mole. In alternate non-limiting embodiments, the hydroxyl-containing material can have a number average molecular weight of less than 10,000 grams/mole, or less than 15,000 grams/mole, or less than 20,000 grams/mole, or less than 32,000 grams/mole.

In a non-limiting embodiment, the polyether-containing polyol material for use in the present invention can include teresters produced from at least one low molecular weight dicarboxylic acid, such as adipic acid.

Polyether glycols for use in the present invention can include but are not limited to polytetramethylene ether glycol.

In a non-limiting embodiment, the active hydrogen-containing material can comprise block polymers including blocks of ethylene oxide-propylene oxide and/or ethylene oxide-butylene oxide. In a non-limiting embodiment, the active hydrogen-containing material can comprise a block polymer of the following chemical formula:

$$\text{(I'')} \qquad H\text{-}(O\text{-}CRRCRR\text{-}Y_n)_a\text{-}(CRRCRR\text{-}Y_n\text{-}O)_b\text{-}(CRRCRR\text{-}Y_n\text{-}O)_c\text{-}H$$

wherein R can represent hydrogen or $C_1$-$C_6$ alkyl; $Y_n$ can represent $C_0$ -$C_6$ hydrocarbon; n can be an integer from 0 to 6; a, b, and c can each be an integer from 0 to 300, wherein a, b and c are chosen such that the number average molecular weight of the polyol does not exceed 32,000 grams/mole.

In a further non-limiting embodiment, Pluronic R, Pluronic L62D, Tetronic R and Tetronic, which are commercially available from BASF, can be used as the active hydrogen-containing material in the present invention.

Non-limiting examples of suitable polyols for use in the present invention include straight or branched chain alkane polyols, such as but not limited to 1,2-ethanediol, 1,3-propanediol, 1,2-propanediol, 1,4-butanediol, 1,3-butanediol, glycerol, neopentyl glycol, trimethylolethane, trimethylolpropane, di-trimethylolpropane, erythritol, pentaerythritol and dipentaerythritol; polyalkylene glycols, such as but not limited to diethylene glycol, dipropylene glycol and higher polyalkylene glycols such as but not limited to polyethylene glycols which can have number average molecular weights of from 200 grams/mole to 2,000 grams/mole; cyclic alkane polyols, such as but not limited to cyclopentanediol, cyclohexanediol, cyclohexanetriol, cyclohexanedimethanol, hydroxypropylcyclohexanol and cyclohexanediethanol; aromatic polyols, such as but not limited to dihydroxybenzene, benzenetriol, hydroxybenzyl alcohol and dihydroxytoluene; bisphenols, such as, 4,4'-isopropylidenediphenol; 4,4'-oxybisphenol, 4,4'-dihydroxybenzophenone, 4,4'-thiobisphenol, phenolphthlalein, bis(4-hydroxyphenyl)methane, 4,4'-(1,2-ethenediyl)bisphenol and 4,4'-sulfonylbisphenol; halogenated bisphenols, such as but not limited to 4,4'-isopropylidenebis(2,6-dibromophenol), 4,4'-isopropylidenebis(2,6-dichlorophenol) and 4,4'-isopropylidenebis(2,3,5,6-tetrachlorophenol); alkoxylated bisphenols, such as but not limited to alkoxylated 4,4'-isopropylidenediphenol which can have from 1 to 70 alkoxy groups, for example, ethoxy, propoxy, $\alpha$-butoxy and $\beta$-butoxy groups; and biscyclohexanols, which can be prepared by hydrogenating the corresponding bisphenols, such as but not limited to 4,4'-isopropylidene-biscyclohexanol, 4,4'-oxybiscyclohexanol, 4,4'-thiobiscyclohexanol and bis(4-hydroxycyclohexanol)methane; polyurethane polyols, polyester polyols, polyether polyols, poly vinyl alcohols, polymers containing hydroxy functional acrylates, polymers containing hydroxy functional methacrylates, and polymers containing allyl alcohols.

In a non-limiting embodiment, the polyol can be chosen from multifunctional polyols, including but not limited to trimethylolpropane, ethoxylated trimethylolpropane, pentaerythritol.

In a further non-limiting embodiment, the polyol can be a polyurethane prepolymer having two or more hydroxy functional groups. Such polyurethane prepolymers can be prepared from any of the above-listed polyols and aforementioned polyisocyanates. In a non-limiting embodiment, the OH:NCO equivalent ratio can be chosen such that essentially no free NCO groups are produced in preparing the polyurethane prepolymer. In a non-limiting embodiment, the equivalent ratio of NCO (i.e., isocyanate) to OH present in the polyether-containing polyurethane prepolymer can be an amount of from 2.0 to less than 5.5 NCO/1.0 OH.

In alternate non-limiting embodiments, the polyurethane prepolymer can have a number average molecular weight (Mn) of less than 50,000 grams/mole, or less than 20,000 grams/mole, or less than 10,000 grams/mole. The Mn can be determined using a variety of known methods. In a non-limiting embodiment, the Mn can be determined by gel permeation chromatography (GPC) using polystyrene standards.

In a non-limiting embodiment, the sulfur-containing active hydrogen material for use in the present invention can include a SH-containing material such as but not limited to a polythiol having at least two thiol groups. Non-limiting examples of suitable polythiols can include but are not limited to aliphatic polythiols, cycloaliphatic polythiols, aromatic polythiols, heterocyclic polythiols, polymeric polythiols and mixtures thereof. The hydrogen-containing material can have linkages including but not limited to ether linkages (-O-), sulfide linkages (-S-), polysulfide linkages ($-S_x-$, wherein x is at least 2, or from 2 to 4) and combinations of such linkages. As used herein and the claims, the terms "thiol," "thiol group," "mercapto" or "mercapto group" refer to an -SH group which is capable of forming a thiourethane linkage, (i.e., -NH-C(O)-S-) with an isocyanate group or a dithioruethane linkage (i.e., -NH-C(S)-S-) with an isothiocyanate group.

Non-limiting examples of suitable polythiols can include but are not limited to 2,5-dimercaptomethyl-1,4-dithiane, dimercaptoethylsulfide, pentaerythritol tetrakis(3-mercaptopropionate), pentaerythritol tetrakis(2-mercaptoacetate), trimethylolpropane tris(3-mercaptopropionate), trimethylolpropane tris(2-mercaptoacetate), 4-mercaptomethyl-3,6-dithia-1,8-octanedithiol, 4-tert-butyl-1,2-benzenedithiol, 4,4'-thiodibenzenethiol, ethanedithiol, benzenedithiol, ethylene glycol di(2-mercaptoacetate), ethylene glycol di(3-mercaptopropionate), poly(ethylene glycol) di(2-mercaptoacetate) and poly(ethylene glycol) di(3-mercaptopropionate), and mixtures thereof.

In a non-limiting embodiment, the polythiol can be chosen from materials represented by the following general formula,

(II)

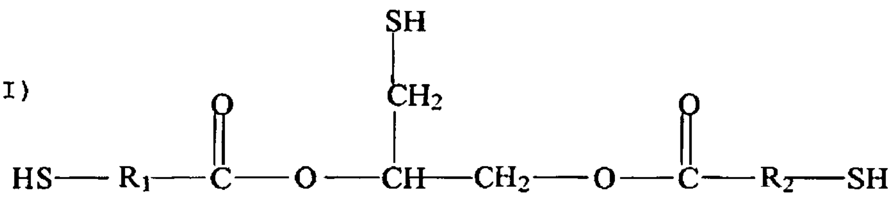

wherein $R_1$ and $R_2$ can each be independently chosen from straight or branched chain alkylene, cyclic alkylene, phenylene and $C_1$-$C_9$ alkyl substituted phenylene. Non-limiting examples of straight or branched chain alkylene can include but are not limited to methylene, ethylene, 1,3-propylene, 1,2-propylene, 1,4-butylene, 1,2-butylene, pentylene, hexylene, heptylene, octylene, nonylene, decylene, undecylene, octadecylene and icosylene. Non-limiting examples of cyclic alkylenes can include but are not limited to cyclopentylene, cyclohexylene, cycloheptylene, cyclooctylene, and alkyl-substituted derivatives thereof. In a non-limiting embodiment, the divalent linking groups $R_1$ and $R_2$ can be chosen from phenylene and alkyl-substituted phenylene, such as methyl, ethyl, propyl, isopropyl and nonyl substituted phenylene. In a further non-limiting embodiment, $R_1$ and $R_2$ are each methylene or ethylene.

The polythiol represented by general formula II can be prepared by any known method. In a non-limiting embodiment, the polythiol of formula (II) can be prepared from an esterification or transesterification reaction between 3-mercapto-1,2-propanediol (Chemical Abstract Service (CAS) Registry No. 96-27-5) and a thiol functional carboxylic acid or carboxylic acid ester in the presence of a strong acid catalyst, such as but not limited to methane sulfonic acid, with the concurrent removal of water or alcohol from the reaction mixture. A non-limiting example of a polythiol of formula II includes a structure wherein $R_1$ and $R_2$ are each methylene.

In a non-limiting embodiment, the polythiol represented by general formula II can be thioglycerol bis(2-mercaptoacetate). As used herein and the claims, the term "thioglycerol bis(2-mercaptoacetate)" refers to any related co-product oligomeric species and polythiol monomer compositions containing residual starting materials. In a non-limiting embodiment, oxidative coupling of thiol groups can occur when washing the reaction mixture resulting from the esterification of 3-mercapto-1,2-propanediol and a thiol functional carboxylic acid, such as but not limited to 2-mercaptoacetic acid, with excess base, such as but not limited to aqueous ammonia. Such an oxidative coupling can result in the formation of oligomeric polythiol species having disulfide linkages, such as but not limited to -S-S-linkages.

Suitable polythiols for use in the present invention can include but are not limited to polythiol oligomers having disulfide linkages, which can be prepared from the reaction of a polythiol having at least two thiol groups and sulfur in the presence of a basic catalyst. In a non-limiting embodiment, the equivalent ratio of polythiol monomer to sulfur can be from m to (m-1) wherein m can represent an integer from 2 to 21. The polythiol can be chosen from the above-mentioned examples, such as but not limited to 2,5-dimercaptomethyl-1,4-dithiane. In alternate non-limiting embodiments, the sulfur can be in the form of crystalline, colloidal, powder and sublimed sulfur, and can have a purity of at least 95 percent or at least 98 percent.

Non-limiting examples of co-product oligomeric species can include materials represented by the following

general formula:

(III)

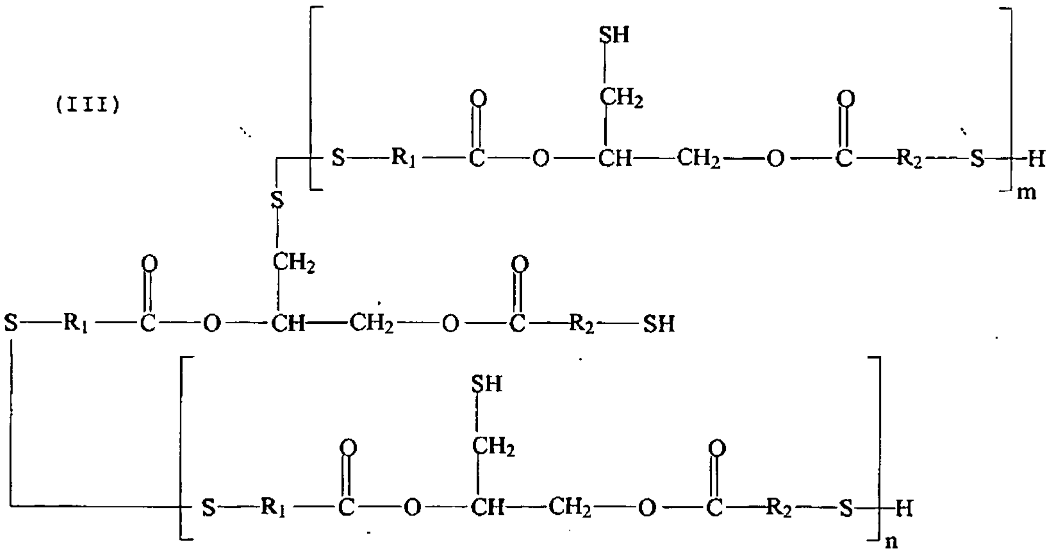

wherein $R_1$ and $R_2$ can be as described above, n and m can be independently an integer from 0 to 21 and (n + m) can be at least 1.

In another non-limiting embodiment, the polythiol oligomer can have disulfide linkages and can include materials represented by the following general formula IV,

(IV)

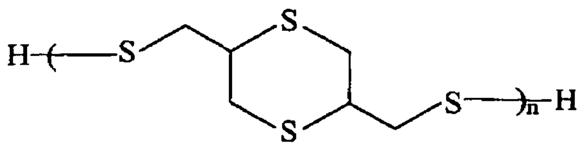

wherein n can represent an integer from 1 to 21. In a non-limiting embodiment, the polythiol oligomer represented by general formula IV can be prepared by the reaction of 2,5-dimeracaptomethyl-1,4-dithiane with sulfur in the presence of a basic catalyst, as described previously herein.

In a non-limiting embodiment, the polythiol for use in the present invention, can include at least one polythiol represented by the following structural formulas.

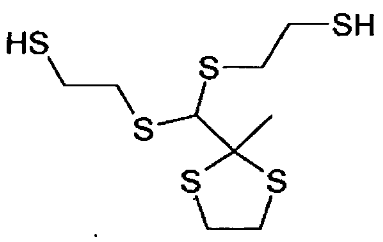

(IV'a)

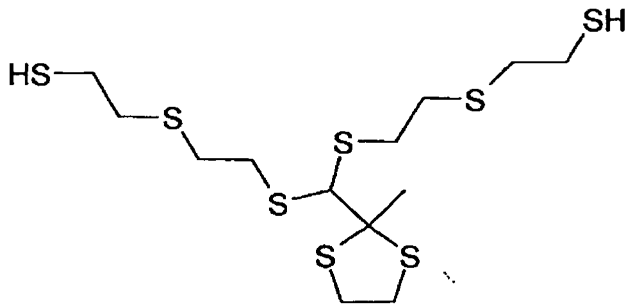

(IV'b)

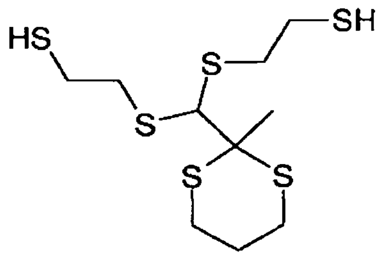

(IV'c)

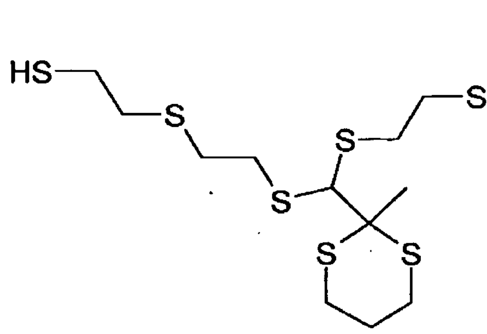

(IV'd)

The sulfide-containing polythiols comprising 1,3-dithiolane (e.g., formulas IV'a and b) or 1,3-dithiane (e.g., formulas IV'c and d) can be prepared by reacting asym-dichloroacetone with polymercaptan, and then reacting the reaction product with polymercaptoalkylsulfide, polymercaptan or mixtures thereof.

Non-limiting examples of suitable polymercaptans for use in the reaction with asym-dichloroacetone can include but are not limited to materials represented by the following formula,

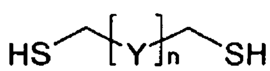

1

wherein Y can represent $CH_2$ or ($CH_2$-S-$CH_2$), and n can be an integer from 0 to 5. In a non-limiting embodiment, the polymercaptan for reaction with asym-dichloroacetone in the present invention can be chosen from ethanedithiol, propanedithiol, and mixtures thereof.

The amount of asym-dichloroacetone and polymercaptan suitable for carrying out the above reaction can vary. In a non-limiting embodiment, asym-dichloroacetone and polymercaptan can be present in the reaction mixture in an amount such that the molar ratio of dichloroacetone to polymercaptan can be from 1:1 to 1:10.

Suitable temperatures for reacting asym-dichloroacetone with polymercaptane can vary. In a non-limiting embodiment, the reaction of asym-dichloroacetone with polymercaptane can be carried out at a temperature within the range of from 0 to 100°C.

Non-limiting examples of suitable polymercaptans for use in the reaction with the reaction product of the asym-dichloroacetone and polymercaptan, can include but are not limited to materials represented by the above general formula 1, aromatic polymercaptans, cycloalkyl polymercaptans, heterocyclic polymercaptans, branched polymercaptans, and mixtures thereof.

Non-limiting examples of suitable polymercaptoalkylsulfides for use in the reaction with the reaction product of the asym-dichloroacetone and polymercaptan, can include but are not limited to materials represented by the following formula,

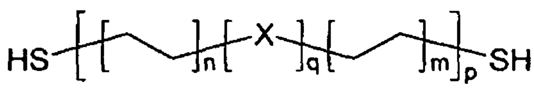

2

wherein X can represent O, S or Se, n can be an integer from 0 to 10, m can be an integer from 0 to 10, p can be an integer from 1 to 10, q can be an integer from 0 to 3, and with the proviso that (m + n) is an integer from 1 to 20.

Non-limiting examples of suitable polymercaptoalkylsulfides for use in the present invention can include branched polymercaptoalkylsulfides. In a non-limiting embodiment, the polymercaptoalkylsulfide for use in the present invention can be dimercaptoethylsulfide.

The amount of polymercaptan, polymercaptoalkylsulfide, or mixtures thereof, suitable for reacting with the reaction product of asym-dichloroacetone and polymercaptan, can vary. In a non-limiting embodiment, polymercaptan, polymercaptoalkylsulfide, or a mixture thereof, can be present in the reaction mixture in an amount such that the equivalent ratio of reaction product to polymercaptan, polymercaptoalkylsulfide, or a mixture thereof, can be from 1:1.01 to 1:2.

Moreover, suitable temperatures for carrying out this reaction can vary. In a non-limiting embodiment, the reaction of polymercaptan, polymercaptoalkylsulfide, or a mixture thereof, with the reaction product can be carried out at a temperature within the range of from 0 to 100°C.

In a non-limiting embodiment, the reaction of asym-dichloroacetone with polymercaptan can be carried out in the presence of an acid catalyst. The acid catalyst can be selected from a wide variety known in the art, such as but not limited to Lewis acids and Bronsted acids. Non-limiting examples of suitable acid catalysts can include those described in Ullmann's Encyclopedia of Industrial Chemistry, 5th Edition, 1992, Volume A21, pp. 673 to 674. In further alternate non-limiting embodiments, the acid catalyst can be chosen from boron trifluoride etherate, hydrogen chloride, toluenesulfonic acid, and mixtures thereof.

The amount of acid catalyst can vary. In a non-limiting embodiment, a suitable amount of acid catalyst can be from 0.01 to 10 percent by weight of the reaction mixture.

In another non-limiting embodiment, the reaction product of asym-dichloroacetone and polymercaptan can be reacted with polymercaptoalkylsulfide, polymercaptan or mixtures thereof, in the presence of a base. The base can be selected from a wide variety known in the art, such as but not limited to Lewis bases and Bronsted bases. Non-limiting examples of suitable bases can include those described in Ullmann's Encyclopedia of Industrial Chemistry, 5th Edition, 1992, Volume A21, pp. 673 to 674. In a further non-limiting embodiment, the base can be sodium hydroxide.

The amount of base can vary. In a non-limiting embodiment, a suitable equivalent ratio of base to reaction product of the first reaction, can be from 1:1 to 10:1.

In another non-limiting embodiment, the preparation of these sulfide-containing polythiols can include the use of a solvent. The solvent can be selected from a wide variety known in the art.

In a further non-limiting embodiment, the reaction of asym-dichloroacetone with polymercaptan can be carried out in the presence of a solvent. The solvent can be selected from a wide variety of known materials. In a non-limiting embodiment, the solvent can be selected from but is not limited to organic solvents, including organic inert solvents. Non-limiting examples of suitable solvents can include but are not limited to chloroform, dichloromethane, 1,2-dichloroethane, diethyl ether, benzene, toluene, acetic acid and mixtures therof. In still a further embodiment, the reaction of asym-dichloroacetone with polymercaptan can be carried out in the presence of toluene as solvent.

In another embodiment, the reaction product of asym-dichloroacetone and polymercaptan can be reacted with polymercaptoalkylsulfide, polymercaptan or mixtures thereof, in the presence of a solvent, wherein the solvent can be selected from but is not limited to organic solvents including organic inert solvents. Non-limiting examples of suitable organic and inert solvents can include alcohols such as but not limited to methanol, ethanol and propanol; aromatic hydrocarbon solvents such as but not limited to benzene, toluene, xylene; ketones such as but not limited to methyl ethyl ketone; water and mixtures thereof. In a further non-limiting embodiment, this reaction can be carried out in the presence of a mixture of toluene and water as solvent system. In another non-limiting embodiment, this reaction can be carried out in the presence of ethanol as solvent.

The amount of solvent can widely vary. In a non-limiting embodiment, a suitable amount of solvent can be from 0 to 99 percent by weight of the reaction mixture. In a further non-limiting embodiment, the reaction can be carried out neat, i.e., without solvent.

In another non-limiting embodiment, the reaction of asym-dichloroacetone with polyercaptan can be carried out in the presence of a dehydrating reagent. The dehydrating reagent can be selected from a wide variety known in the art. Suitable dehydrating reagents for use in this reaction can include but are not limited to magnesium sulfate. The amount of dehydrating reagent can vary widely according to the stoichiometry of the dehydrating reaction.

In a non-limiting embodiment, a sulfide-containing polythiol of the present invention can be prepared by reacting 1,1-dichloroacetone with 1,2-ethanedithiol to produce 2-methyl-2-dichloromethyl-1,3-dithiolane, as shown below.

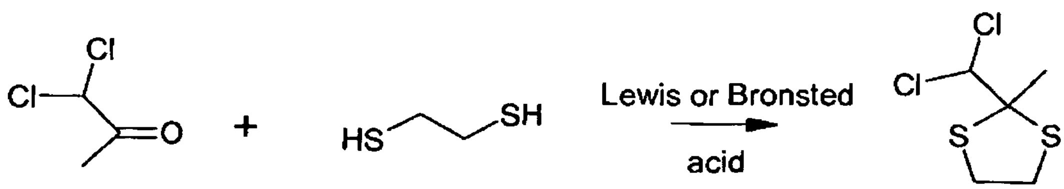


In a further non-limiting embodiment, 1,1-dichloroacetone can be reacted with 1,3-propanedithiol to produce a 2-methyl-2-dichloromethyl-1,3-dithiane, as shown below.

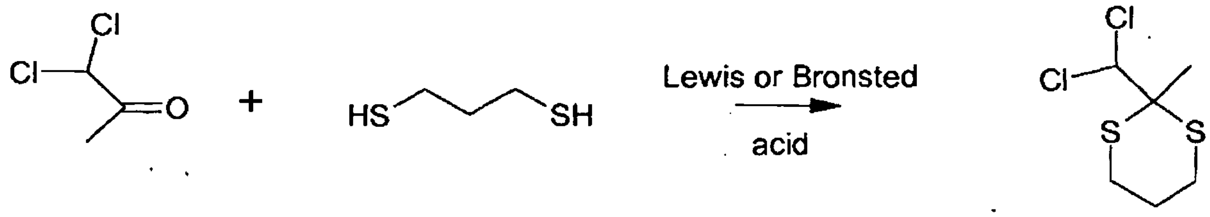


In another non-limiting embodiment, 2-methyl-2-dichloromethyl-1,3-dithiolane can be reacted with dimercaptoethylsulfide to produce a dimercapto 1,3-dithiolane derivative of the present invention, as shown below.

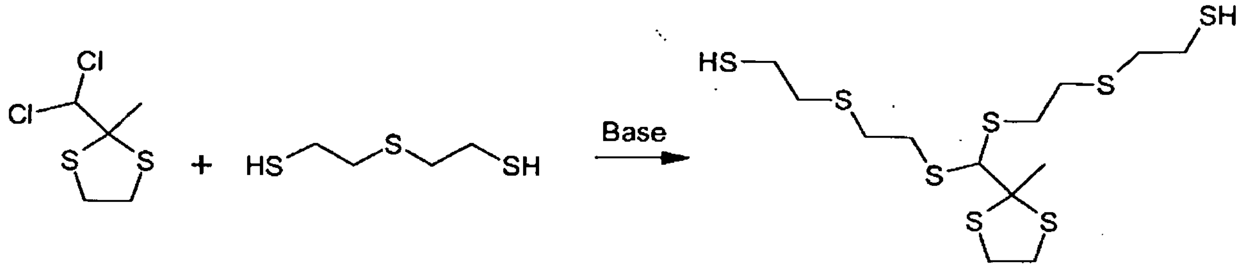


In another non-limiting embodiment, 2-methyl-2-dichloromethyl-1,3-dithiolane can be reacted with 1,2-ethanedithiol to produce a dimercapto 1,3-dithiolane derivative of the present invention, as shown below.

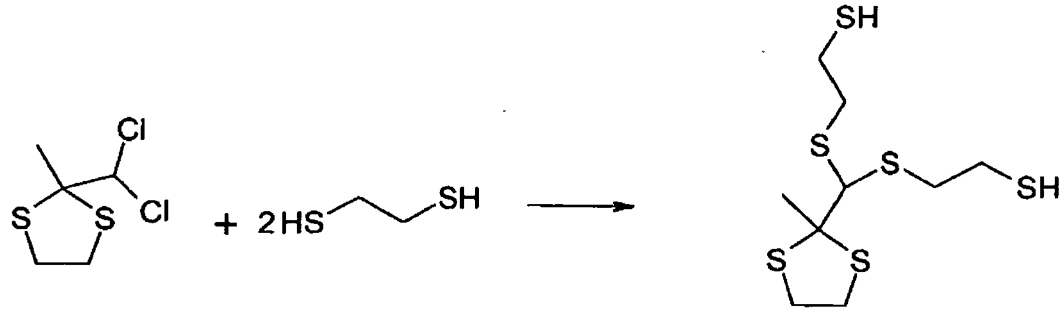

In another non-limiting embodiment, 2-methyl-2-dichloromethyl-1,3-dithiane can be reacted with dimercaptoethylsulfide to produce a dimercapto 1,3-dithiane derivative of the present invention as shown below.

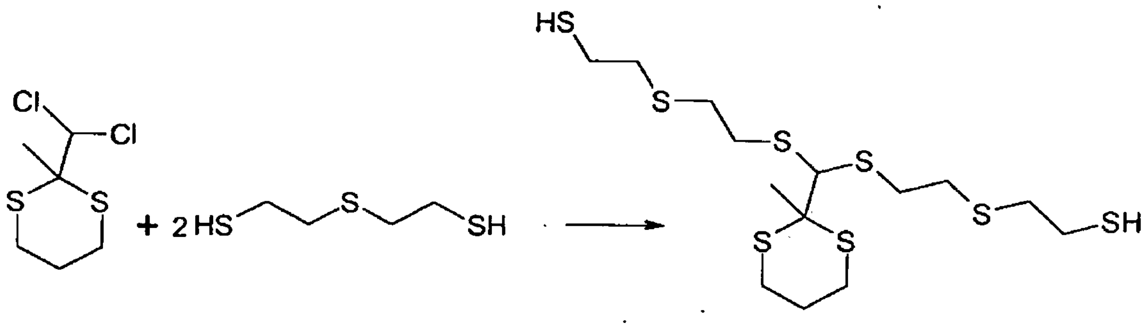

In another non-limiting embodiment, 2-methyl-2-dichloromethyl-1,3-dithiane can be reacted with 1,2-ethanedithiol to produce a dimercapto 1,3-dithiane derivative of the present invention as shown below.

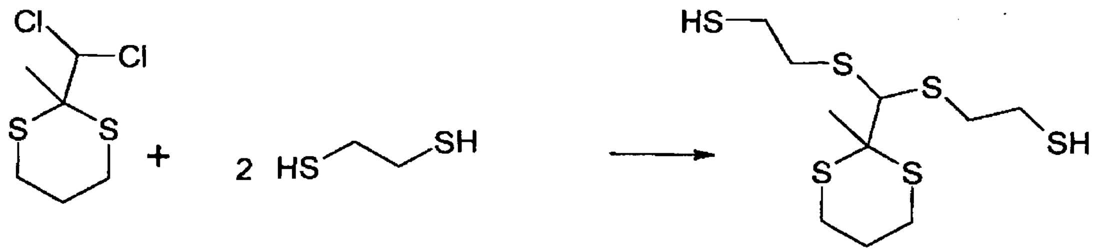

In another non-limiting embodiment, the polythiol for use in the present invention can include at least one oligomeric polythiol prepared by reacting an asym-dichloro derivative with a polymercaptoalkylsulfide as follows.

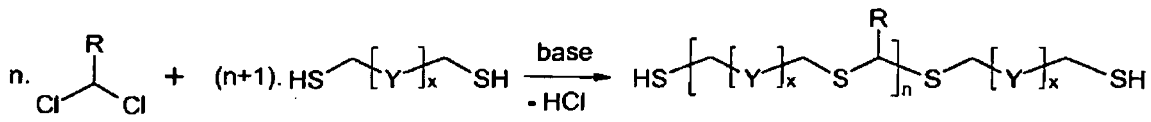


(IV'e)

wherein R can represent $CH_3$, $CH_3CO$, $C_1$ to $C_{10}$ alkyl, cycloalkyl, aryl alkyl, or alkyl-CO; Y can represent $C_1$ to $C_{10}$ alkyl, cycloalkyl, $C_6$ to $C_{14}$ aryl, $(CH_2)_p(S)_m(CH_2)_q$, $(CH_2)_p(Se)_m(CH_2)_q$, $(CH_2)_p(Te)_m(CH_2)_q$ wherein m can be an integer from 1 to 5 and, p and q can each be an integer from 1 to 10; n can be an integer from 1 to 20; and x can be an integer from 0 to 10.

In a further non-limiting embodiment, a polythioether oligomeric dithiol can be prepared by reacting asym-dichloroacetone with polymercaptoalkylsulfide, in the presence of a base. Non-limiting examples of suitable polymercaptoalkylsulfides for use in this reaction can include but are not limited to those materials represented by general formula 2 as previously recited herein. Suitable bases for use in this reaction can include those previously recited herein.

Further non-limiting examples of suitable polymercaptoalkylsulfides for use in the present invention can include branched polymercaptoalkylsulfides. In a non-limiting embodiment, the polymercaptoalkylsulfide can be dimercaptoethylsulfide.

In a non-limiting embodiment, the reaction of asymdichloro derivative with polymercaptoalkylsulfide can be carried out in the presence of a base. Non-limiting examples of suitable bases can include those previously recited herein.

In another non-limiting embodiment, the reaction of asym-dichloro derivative with polymercaptoalkylsulfide can be carried out in the presence of a phase transfer catalyst. Suitable phase transfer catalysts for use in the present invention are known and varied. Non-limiting examples can include but are not limited to tetraalkylammonium salts and tetraalkylphosphonium salts. In a further non-limiting embodiment, this reaction can be carried out in the presence of tetrabutylphosphonium bromide as phase transfer catalyst. The amount of phase transfer catalyst can vary widely. In a non-limiting embodiment, the amount of phase transfer catalyst can be from 0 to 50 equivalent percent, or from 0 to 10 equivalent percent, or from 0 to 5 equivalent percent, to the polymercaptosulfide reactants.

In another non-limiting embodiment, the preparation of the polythioether oligomeric dithiol can include the use of solvent. Non-limiting examples of suitable solvents can include those previously recited herein.

In a non-limiting embodiment, "n" moles of 1,1-dichloroacetone can be reacted with "n+1" moles of polymercaptoethylsulfide wherein n can represent an integer of from 1 to 20, to produce a polythioether oligomeric dithiol as follows.

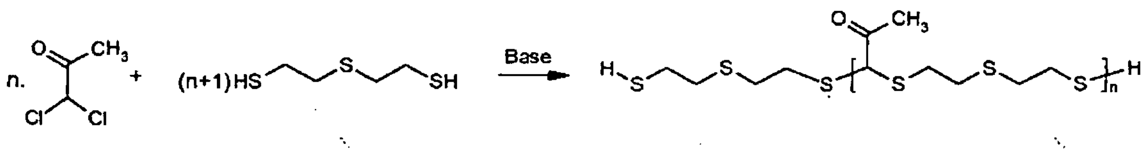


In a further non-limiting embodiment, a polythioether oligomeric dithiol of the present invention can be prepared by introducing "n" moles of 1,1-dichloroethane together with "n+1" moles of polymercaptoethylsulfide as follows,

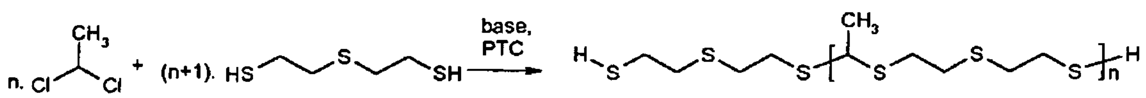

wherein n can represent an integer from 1 to 20.

In alternate non-limiting embodiments, the polythiol for use in the present invention can include at least one oligomeric polythiol represented by the following structural formulas and prepared by the following methods.

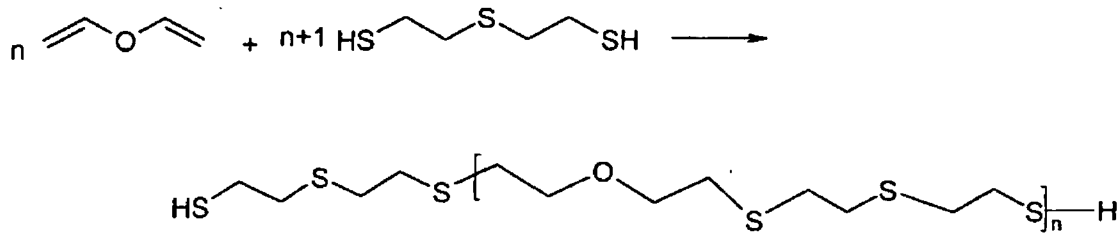

(IV'f)

wherein n can be an integer from 1 to 20.

Various methods of preparing the polythiol of formula (IV'f) are described in detail in United States Patent 6,509,418B1, column 4, line 52 through column 8, line 25. In general, this polythiol can be prepared by reacting reactants comprising one or more polyvinyl ether monomer, and one or more polythiol material. Useful polyvinyl ether monomers can include but are not limited to divinyl ethers represented by structural formula (V'):

$$CH_2{=}CH\text{--}O\text{--}(\text{--}R^2\text{--}O\text{--})_m\text{--}CH{=}CH_2 \qquad (V')$$

wherein $R_2$ can be $C_2$ to $C_6$ n-alkylene, $C_2$ to $C_6$ branched alkylene, $C_6$ to $C_8$ cycloalkylene or $C_6$ to $C_{10}$ alkylcycloalkylene group or $\text{--}[(CH_2\text{--})_p\text{--}O\text{--}]_q\text{--}(\text{--}CH_2\text{--})_r\text{--}$, and m is a rational number ranging from 0 to 10, p is an independently selected integer ranging from 2 to 6, q is an independently selected integer ranging from 1 to 5 and r is an independently selected integer ranging from 2 to 10.

In a non-limiting embodiment, m can be zero (0).

Non-limiting examples of suitable polyvinyl ether monomers for use can include divinyl ether monomers, such as but not limited to divinyl ether and ethylene glycol divinyl ether.

In alternate non-limiting embodiments, the polyvinyl ether monomer can include from 20 to less than 50 mole percent of the reactants used to prepare the polythiol, or from 30 to less than 50 mole percent.

The divinyl ether of formula (V') can be reacted with a polythiol such as but not limited to a dithiol having the formula (VI'):

HS--R1 --SH (VI')

wherein R1 can be a C2 to C6 n-alkylene group; C3 to C6 branched alkylene group, having one or more pendant groups which can include but are not limited to, hydroxyl groups, alkyl groups such as methyl or ethyl groups; alkoxy groups, or C6 to C8 cycloalkylene.

Non-limiting examples of suitable polythiols can include but are not limited to dithiols such as 1,2-ethanedithiol, 1,2-propanedithiol, 1,3-propanedithiol, 1,3-butanedithiol, 1,4-butanedithiol, 2,3-butanedithiol, 1,3-pentanedithiol, 1,5-pentanedithiol, 1,6-hexanedithiol, 1,3-dimercapto-3-methylbutane, dipentenedimercaptan, ethylcyclohexyldithiol (ECHDT), dimercaptodiethylsulfide, methyl-substituted dimercaptodiethylsulfide, dimethyl-substituted dimercaptodiethylsulfide, dimercaptodioxaoctane, 1,5-dimercapto-3-oxapentane and mixtures thereof. In a non-limiting embodiment, the polythiol of formula (VI') can be dimercaptodiethylsulfide (DMDS).

In a non-limiting embodiment, the polythiol material can have a number average molecular weight ranging from 90 to 1000 grams/mole, or from 90 to 500 grams/mole. In a further non-limiting embodiment, the stoichiometric ratio of polythiol to divinyl ether materials can be less than one equivalent of polyvinyl ether to one equivalent of polythiol.

In a non-limiting embodiment, the reactants can further include one or more free radical catalysts. Non-limiting examples of suitable free radical catalysts can include azo compounds, such as azobis-nitrile compounds such as but not limited to azo(bis)isobutyronitrile (AIBN); organic peroxides such as but not limited to benzoyl peroxide and t-butyl peroxide; inorganic peroxides and similar free-radical generators.

In alternate non-limiting embodiments, the reaction can be effected by irradiation with ultraviolet light either with or without a cationic photoinitiating moiety.

In a non-limiting embodiment, the polythiol for use in the present invention can include a material having the following structural formula:

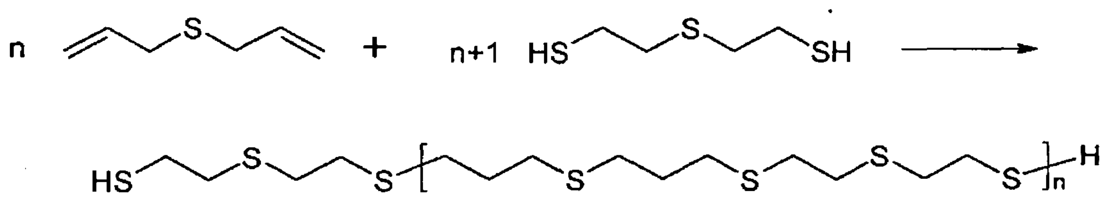

(IV'g)

wherein n can be an integer from 1 to 20.

Various methods of preparing the polythiol of the formula (IV'g) are described in detail in WO 03/042270, page 2, line 16 to page 10, line 7. In general, the polythiol can be a prepolymer having number average molecular weight ranging from 100 to 3000 grams/mol, the prepolymer being free from disulfide (-S-S-) linkage, and can be prepared by ultraviolet (UV) catalyzed free radical polymerization in the presence of a suitable photoinitiator. Suitable photoinitiators in usual amounts as known to one skilled in the art can be used for this process. In a non-limiting embodiment, 1-hydroxycyclohexyl phenyl ketone (Irgacure 184) can be used in an amount of from 0.05% to 0.10% by weight, based on the total weight of the polymerizable monomers present in the mixture.

In a non-limiting embodiment, the polythiol of the formula (IV'g) can be prepared by reacting "n" moles of an allyl sulfide and "n+1" moles of dimercaptodiethylsulfide as shown above.

In a non-limiting embodiment, the polythiol for use in the present invention can include a material represented by the following structural formula:

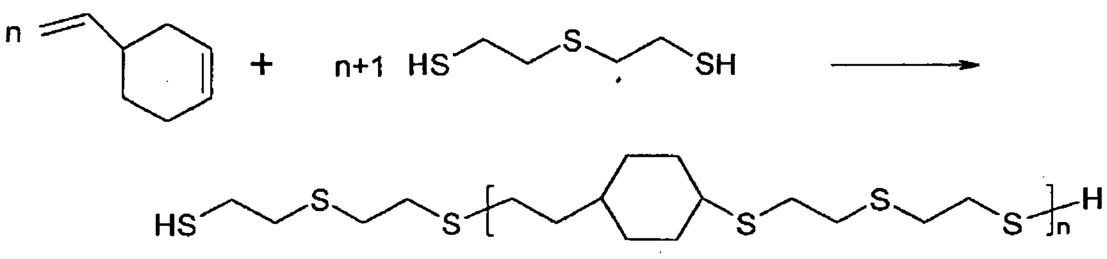

(IV'h)

wherein n can be an integer from 1 to 20.

Various methods for preparing the polythiol of formula (IV'h) are described in detail in WO/01/66623A1, from page 3, line 19 to page 6, line 11. In general, this polythiol can be prepared by the reaction of a thiol such as a dithiol, and an aliphatic, ring-containing non-conjugated diene in the presence of a catalyst. Non-limiting examples of suitable thiols for use in the reaction can include but are not limited to lower alkylene thiols such as ethanedithiol, vinylcyclohexyldithiol, dicyclopentadienedithiol, dipentene dimercaptan, and hexanedithiol; aryl thiols such as benzene dithiol; polyol esters of thioglycolic acid and thiopropionic acid.

Non-limiting examples of suitable cyclodienes can include but are not limited to vinylcyclohexene, dipentene, dicyclopentadiene, cyclododecadiene, cyclooctadiene, 2-cyclopenten-1-yl-ether, 5-vinyl-2-norbornene and norbornadiene.

Non-limiting examples of suitable catalysts for the reaction can include azo or peroxide free radical initiators such as the azobisalkylenenitrile commercially available from DuPont under the trade name VAZO™.

In a further non-limiting embodiment, dimercaptoethylsulfide can be reacted with 4-vinyl-1-cyclohexene, as shown above, with VAZO-52 catalyst.

In another non-limiting embodiment, the polythiol for use in the present invention can include a material represented by the following structural formula:

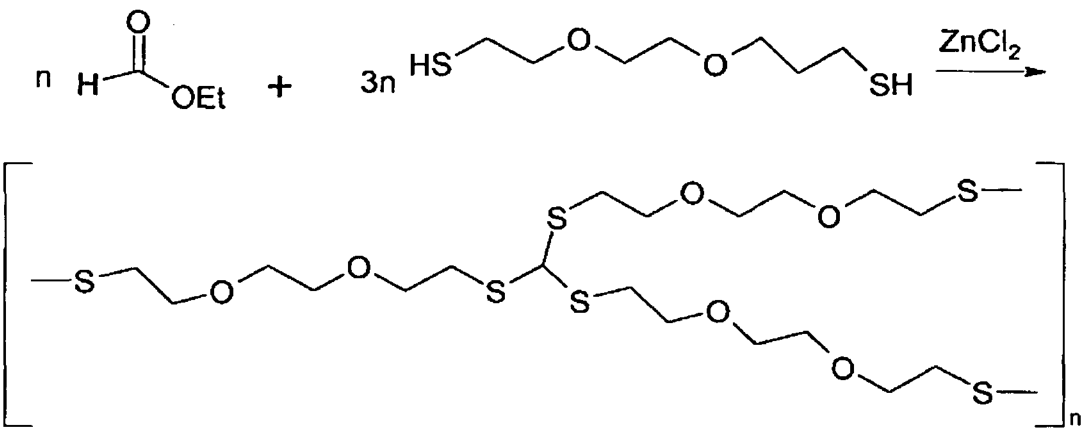


(IV'i)

wherein n can be an integer from 1 to 20.

Various methods of preparing the polythiol of the formula (IV'1) are described in detail in United States Patent 5,225,472, from column 2, line 8 to column 5, line 8.

In a non-limiting embodiment, 1,8-dimercapto-3,6-dioxaooctane (DMDO) can be reacted with ethyl formate, as shown above, in the presence of anhydrous zinc chloride.

In alternate non-limiting embodiments, the active hydrogen-containing material for use in the present invention can be chosen from polyether glycols and polyester glycols having a number average molecular weight of at least 200 grams/mol, or at least 300 grams/mol, or at least 750 grams/mol; or no greater than 1,500 grams/mol, or no greater than 2,500 grams/mol, or no greater than 4,000 grams/mol.

Non-limiting examples of suitable materials having both hydroxyl and thiol groups can include but are not limited to 2-mercaptoethanol, 3-mercapto-1,2-propanediol, glycerin bis(2-mercaptoacetate), glycerin bis(3-mercaptopropionate), 1-hydroxy-4-mercaptocyclohexane, 2,4-dimercaptophenol, 2-mercaptohydroquinone, 4-mercaptophenol, 1,3-dimercapto-2-propanol, 2,3-dimercapto-1-propanol, 1,2-dimercapto-1,3-butanediol, trimethylolpropane bis(2-mercaptoacetate), trimethylolpropane bis(3-mercaptopropionate), pentaerythritol mono(2-mercaptoacetate), pentaerythritol bis(2-mercaptoacetate), pentaerythritol tris(2-mercaptoacetate), pentaerythritol mono(3-mercaptopropionate), pentaerythritol bis(3-mercaptopropionate), pentaerythritol tris(3-mercaptopropionate), hydroxymethyl-tris(mercaptoethylthiomethyl)methane, 1-hydroxyethylthio-3-mercaptoethylthiobenzene, 4-hydroxy-4'-mercaptodiphenylsulfone, dihydroxyethyl sulfide mono(3-mercaptopropionate and hydroxyethylthiomethyl-tris(mercaptoethylthio)methane.

The polycyanate and active hydrogen-containing material are reacted to form a polyurethane prepolymer, and the polyurethane prepolymer is reacted with an amine-containing curing agent.

Amine-containing curing agents for use in the present invention are numerous and widely varied. Non-limiting examples of suitable amine-containing curing agents can include but are not limited to aliphatic polyamines, cycloaliphatic polyamines, aromatic polyamines and mixtures thereof. In alternate non-limiting embodiments, the amine-containing curing agent can be a polyamine having at least two functional groups independently chosen from primary amine ($-NH_2$), secondary amine (-NH-) and combinations thereof. In a further non-limiting embodiment, the amine-containing curing agent can have at least two primary amine groups. The amine-containing curing agent comprises a mixture of a polyamine and at least one material selected from a polythiol and polyol. Non-limiting examples of suitable polythiols and polyols include those previously recited herein. In still another non-limiting embodiment, the amine-containing curing agent can be a sulfur-containing amine-containing curing agent. A non-limiting example of a sulfur-containing amine-containing curing agent can include Ethacure 300 which is commercially available from Albemarle Corporation.

In an embodiment wherein it is desirable to produce a polyureaurethane having low color, the amine-curing agent can be chosen such that it has relatively low color and/or it can be manufactured and/or stored in a manner as to prevent the amine from developing color (e.g., yellow).

Suitable amine-containing curing agents for use in the present invention can include but are not limited to materials having the following chemical formula:

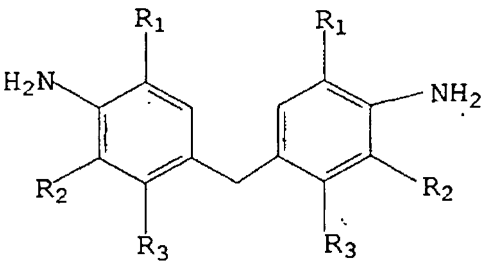

(XII')

wherein $R_1$ and $R_2$ can each be independently chosen from methyl, ethyl, propyl, and isopropyl groups, and $R_3$ can be chosen from hydrogen and chlorine. Non-limiting examples of amine-containing curing agents for use in the present invention include the following compounds, manufactured by Lonza Ltd. (Basel, Switzerland):

LONZACURE.RTM. M-DIPA: $R_1$=$C_3$ $H_7$; $R_2$=$C_3$ $H_7$; $R_3$=H
LONZACURE. RTM. M-DMA: $R_1$=$CH_3$; $R_2$=$CH_3$; $R_3$=H
LONZACURE.RTM. M-MEA: $R_1$=$CH_3$; $R_2$=$C_2$ $H_5$; $R_3$=H
LONZACURE.RTM. M-DEA: $R_1$=$C_2$ $H_5$; $R_2$=$C_2$ $H_5$; $R_3$=H
LONZACURE.RTM. M-MIPA: $R_1$=$CH_3$; $R_2$=$C_3$ $H_7$; $R_3$=H
LONZACURE.RTM. M-CDEA: $R_1$=$C_2$ $H_5$; $R_2$=$C_2$ $H_5$; $R_3$=Cl

wherein $R_1$, $R_2$ and $R_3$ correspond to the aforementioned chemical formula.

In a non-limiting embodiment, the amine-containing curing agent can include but is not limited to a diamine curing agent such as 4,4'-methylenebis(3-chloro-2,6-diethylaniline), (Lonzacure.RTM. M-CDEA), which is available in the United States from Air Products and Chemical, Inc. (Allentown, Pa.). In alternate non-limiting embodiments, the amine-containing curing agent for use in the present invention can include 2,4-diamino-3,5-diethyl-toluene, 2,6-diamino-3,5-diethyl-toluene and mixtures thereof (collectively "diethyltoluenediamine" or "DETDA"), which is commercially available from Albemarle Corporation under the trade name Ethacure 100; dimethylthiotoluenediamine (DMTDA), which is commercially available from Albemarle Corporation under the trade name Ethacure 300; 4,4'-methylene-bis-(2-chloroaniline) which is commercially available from Kingyorker Chemicals under the trade name MOCA. DETDA can be a liquid at room temperature with a viscosity of 156 mPa·s (cPs) at 25°C. DETDA can be isomeric, with the 2,4-isomer range being from 75 to 81 percent while the 2,6-isomer range can be from 18 to 24 percent.

In a non-limiting embodiment, the color stabilized version of Ethacure 100 (i.e., formulation which contains an additive to reduce yellow color), which is available under the name Ethacure 100S may be used in the present invention.

In a non-limiting embodiment, the amine-containing curing agent can act as a catalyst in the polymerization reaction and can be incorporated into the resulting polymerizate.

Non-limiting examples of the amine-containing curing agent can include ethyleneamines. Suitable ethyleneamines can include but are not limited to ethylenediamine (EDA), diethylenetriamine (DETA), triethylenetetramine (TETA), tetraethylenepentamine (TEPA), pentaethylenehexamine (PEHA), piperazine, morpholine, substituted morpholine, piperidine, substituted piperidine, diethylenediamine (DEDA), and 2-amino-1-ethylpiperazine. In alternate non-limiting embodiments, the amine-containing curing agent can be chosen from one or more isomers of $C_1$-$C_3$ dialkyl toluenediamine, such as but not limited to 3,5-dimethyl-2,4-toluenediamine, 3,5-dimethyl-2,6-toluenediamine, 3,5-diethyl-2,4-toluenediamine, 3,5-diethyl-2,6-toluenediamine, 3,5-diisopropyl-2,4-toluenediamine, 3,5-diisopropyl-2,6-toluenediamine, and mixtures thereof. In alternate non-limiting embodiments, the amine-containing curing agent can be methylene dianiline or trimethyleneglycol di(para-aminobenzoate).

In alternate non-limiting embodiments of the present invention, the amine-containing curing agent can include one of the following general structures:

(XIII)

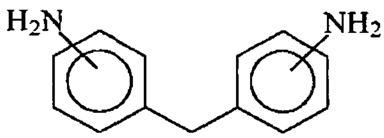

(XIV) 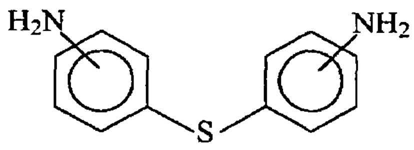

(XV) 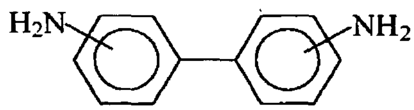

In further alternate non-limiting embodiments, the amine-containing curing agent can include one or more methylene bis anilines which can be represented by the general formulas XVI-XX, one or more aniline sulfides which can be represented by the general formulas XXI-XXV, and/or one or more bianilines which can be represented by the general formulas XXVI-XXVIX,

(XVI) 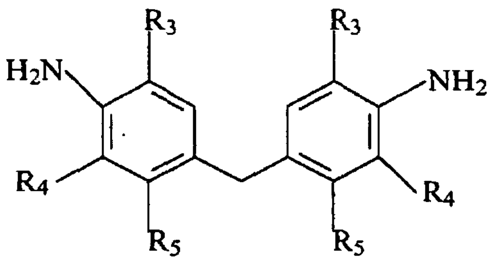

(XVII) 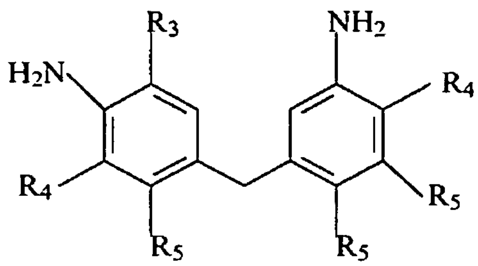

(XVIII) 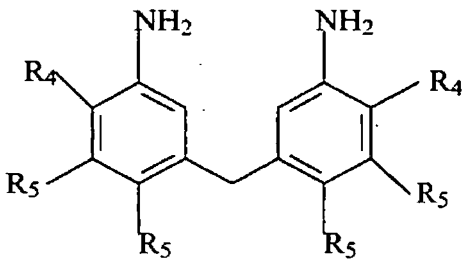

(XIX) 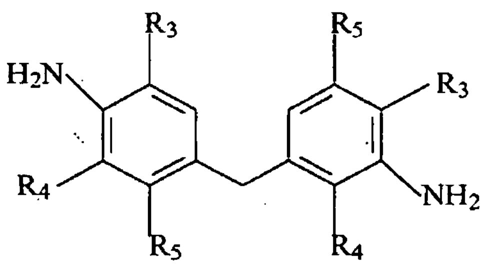

(XX)

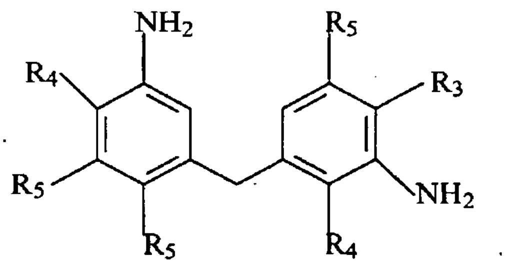

(XXI)

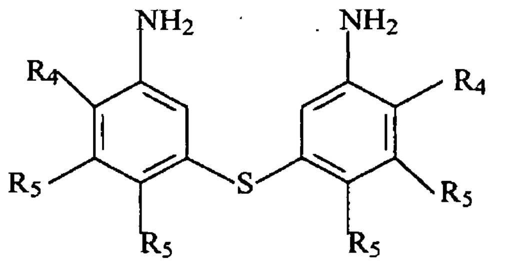

(XXII)

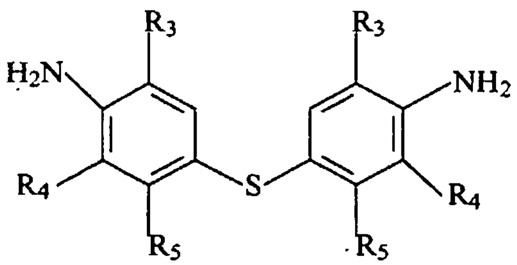

(XXIII)

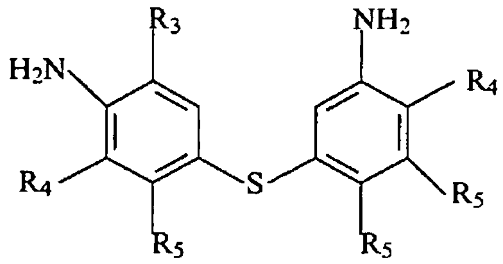

(XXIV)

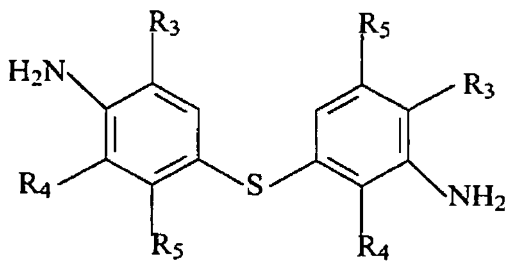

(XXV) 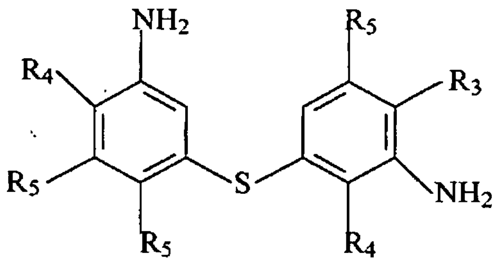

(XXVI) 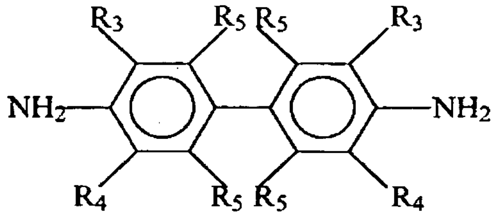

(XXVII) 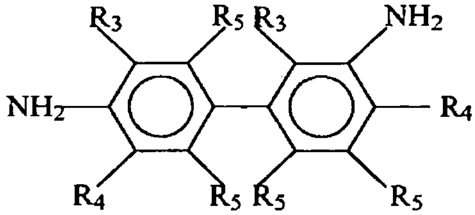

(XXVIII) 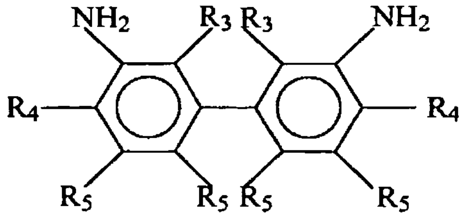

(XXIX) 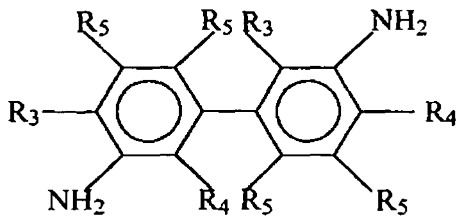

wherein $R_3$ and $R_4$ can each independently represent $C_1$ to $C_3$ alkyl, and $R_5$ can be chosen from hydrogen and halogen, such as but not limited to chlorine and bromine. The diamine represented by general formula XV can be described generally as a 4,4'-methylene-bis(dialkylaniline). Suitable non-limiting examples of diamines which can be represented by general formula XV include but are not limited to 4,4'-methylene-bis(2,6-dimethylaniline), 4,4'-methylene-bis(2,6-diethylaniline), 4,4'-methylene-bis(2-ethyl-6-methylaniline), 4,4'-methylene-bis(2,6-diisopropylaniline), 4,4'-methylene-bis(2-isopropyl-6-methylaniline) and 4,4'-methylene-bis(2,6-diethyl-3-chloroaniline).

In a further non-limiting embodiment, the amine-containing curing agent can include materials which can be represented by the following general structure (XXX):

(XXX) 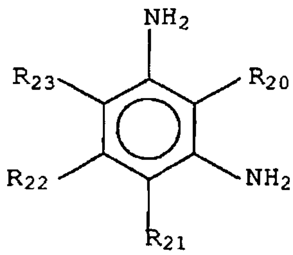

where $R_{20}$, $R_{21}$, $R_{22}$, and $R_{23}$ can be independently chosen from H, $C_1$ to $C_3$ alkyl, $CH_3$-S- and halogen, such as but not limited to chlorine or bromine. In a non-limiting embodiment of the present invention, the amine-containing curing agent which can be represented by general formula XXX can include diethyl toluene diamine (DETDA) wherein $R_{23}$ is methyl, $R_{20}$ and $R_{21}$ are each ethyl and $R_{22}$ is hydrogen. In a further non-limiting embodiment, the amine-containing curing agent can include 4,4'-methylenedianiline.

The sulfur-containing polyureaurethane of the present invention can be polymerized using a variety of techniques known in the art. In a non-limiting embodiment, wherein the polyureaurethane can be prepared by introducing together a polycyanate and an active hydrogen-containing material to form a polyurethane prepolymer and then introducing the amine-containing curing agent, the sulfur-containing polyureaurethane can be polymerized by degassing the prepolymer under vacuum, and degassing the amine-containing curing agent. In a further non-limiting embodiment, these materials can be degassed under vacuum. The amine-containing curing agent can be mixed with the prepolymer using a variety of methods and equipment, such as but not limited to an impeller or extruder.

In another non-limiting embodiment, wherein a lens can be formed, the degassed mixture can be introduced into a mold and the mold can be heated using a variety of conventional techniques known in the art. The thermal cure cycle can vary depending on, for example, the reactivity and molar ratio of the reactants and the presence of catalyst(s). In a non-limiting embodiment, the thermal cure cycle can include heating the prepolymer and curing agent mixture from room temperature to 200°C over a period of from 0.5 hours to 72 hours.

In a non-limiting embodiment, a urethane-forming catalyst can be used in the present invention to enhance the reaction of the polyurethane-forming materials. Suitable urethane-forming catalysts can vary, for example, suitable urethane-forming catalysts can include those catalysts that are useful for the formation of urethane by reaction of the NCO and OH-containing materials, and which have little tendency to accelerate side reactions leading to allophonate and isocyanate formation. Non-limiting examples of suitable catalysts can be chosen from the group of Lewis bases, Lewis acids and insertion catalysts as described in Ullmann's Encyclopedia of Industrial Chemistry, 5th Edition, 1992, Volume A21, pp. 673 to 674. In a non-limiting embodiment, the catalyst can be a stannous salt of an organic acid, such as but not limited to stannous octoate, dibutyl tin dilaurate, dibutyl tin diacetate, dibutyl tin mercaptide, dibutyl tin dimaleate, dimethyl tin diacetate, dimethyl tin dilaurate, 1,4-diazabicyclo[2.2.2]octane, and mixtures thereof. In alternate non-limiting embodiments, the catalyst can be zinc octoate, bismuth, or ferric acetylacetonate.

Further non-limiting examples of suitable catalysts can include tertiary amines such as but not limited to triethylamine, triisopropylamine and N,N-dimethylbenzylamine. Such suitable tertiary amines are disclosed in United States Patent 5,693,738 at column 10, lines 6-38.

In a non-limiting embodiment the catalyst can be chosen from phosphines, tertiary ammonium salts and tertiary amines, such as but not limited to triethylamine; triisopropylamine and N,N-dimethylbenzylamine. Additional non-limiting examples of suitable tertiary amines are disclosed in United States Patent 5,693,738 at column 10 lines 6 through 38.

In a non-limiting embodiment, wherein the sulfur-containing polyureaurethane can be prepared by introducing together a polyurethane prepolymer and an amine-containing curing agent, the polyurethane prepolymer can be reacted with at least one episulfide-containing material prior to being introduced together with amine-containing curing agent. Suitable episulfide-containing materials can vary, and can include but are not limited to materials having at least one, or two, or more episulfide functional groups. In a non-limiting embodiment, the episulfide-containing material can have two or more moieties represented by the following general formula:

(V) 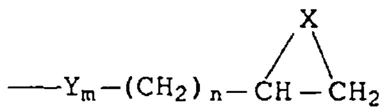

wherein X can be S or O; Y can be $C_1$ - $C_{10}$ alkyl, O, or S; m can be an integer from 0 to 2, and n can be an integer from 0 to 10. In a non-limiting embodiment, the numerical ratio of S is 50% or more, on the average, of the total of S and O constituting a three-membered ring.

The episulfide-containing material having two or more moieties represented by the formula (V) can be attached to an acyclic and/or cyclic skeleton. The acyclic skeleton can be branched or unbranched, and it can contain sulfide and/or ether linkages. In a non-limiting embodiment, the episulfide-containing material can be obtained by replacing the oxygen in an epoxy ring-containing acyclic material using sulfur, thiourea, thiocyanate, triphenylphosphine sulfide or other such reagents known in the art. In a further non-limiting embodiment, alkylsulfide-type episulfide-containing materials can be obtained by reacting various known acyclic polythiols with epichlorohydrin in the presence of an alkali to obtain an alkylsulfide-type epoxy material; and then replacing the oxygen in the epoxy ring as described above.

In alternate non-limiting embodiments, the cyclic skeleton can include the following materials:

(a) an episulfide-containing material wherein the cyclic skeleton can be an alicyclic skeleton,
(b) an episulfide-containing material wherein the cyclic skeleton can be an aromatic skeleton, and
(c) an episulfide-containing material wherein the cyclic skeleton can be a heterocyclic skeleton including a sulfur atom as a hetero-atom.

In further non-limiting embodiments, each of the above materials can contain a linkage of a sulfide, an ether, a sulfone, a ketone, and/or an ester.

Non-limiting examples of suitable episulfide-containing materials having an alicyclic skeleton can include but are not limited to 1,3- and 1,4-bis(β-epithiopropylthio)cyclohexane, 1,3- and 1,4-bis(β-epithiopropylthiomethyl)cyclohexane, bis[4-(β-epithiopropylthio)cyclohexyl] methane, 2,2-bis[4-(β-epithiopropylthio)cyclohexyl] propane, bis[4-(β-epithiopropylthio)cyclohexyl]sulfide, 4-vinyl-1-cyclohexene diepisulfide, 4-epithioethyl-1-cyclohexene sulfide, 4-epoxy-1,2-cyclohexene sulfide, 2,5-bis(β-epithiopropylthio)-1,4-dithiane, and 2,5-bis(β-epithiopropylthioethylthiomethyl)-1,4-dithiane.

Non-limiting examples of suitable episulfide-containing materials having an aromatic skeleton can include but are not limited to 1,3- and 1,4-bis(β-epithiopropylthio)benzene, 1,3- and 1,4-bis(β-epithiopropylthiomethyl)benzene, bis[4-(β-epithiopropylthio)phenyl] methane, 2,2-bis[4-(β-epithiopropylthio)phenyl] propane, bis[4-(β-epithiopropylthio)phenyl] sulfide, bis[4-(β-epithiopropylthio)phenyl] sulfone, and 4,4-bis(β-epithiopropylthio)biphenyl.

Non-limiting examples of suitable episulfide-containing materials having a heterocyclic skeleton including the sulfur atom as the hetero-atom can include but are not limited to the materials represented by the following general formulas:

(VI) 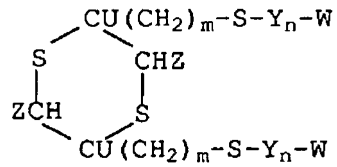

(VII) 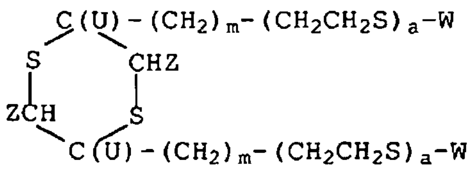

(VII') 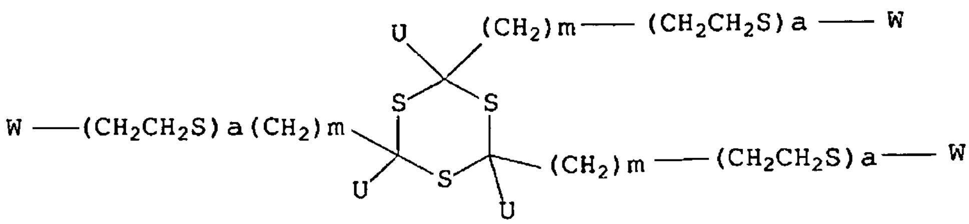

wherein m can be an integer from 1 to 5; n can be an integer from 0 to 4; a can be an integer from 0 to 5; U can be a hydrogen atom or an alkyl group having 1 to 5 carbon atoms; Y can be $-(CH_2CH_2S)-$; Z can be chosen from a hydrogen atom, an alkyl group having 1 to 5 carbon atoms or $-(CH_2)_mSY_nW$; W can be an epithiopropyl group represented by the following formula:

(VIII) 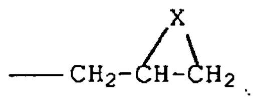

wherein X can be O or S.

Additional non-limiting examples of suitable episulfide-containing materials can include but are not limited to 2,5-bis(β-epithiopropylthiomethyl)-1,4-dithiane; 2,5-bis(β-epithiopropylthioethylthiomethyl)-1,4-dithiane; 2,5-bis(β-epithiopropylthioethyl)-1,4-dithiane; 2,3,5-tri(β-epithiopropylthioethyl)-1,4-dithiane; 2,4,6-tris(β-epithiopropylmethyl)-1,3,5-trithiane; 2,4,6-tris(β-epithiopropylthioethyl)-1,3,5-trithiane; 2,4,6-tris(β-epithiopropylthiomethyl)-1,3,5-trithiane; 2,4,6-tris(β-epithiopropylthioethylthioethyl)-1,3,5-trithiane;

(IX) 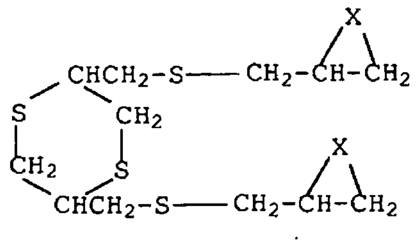

(X) 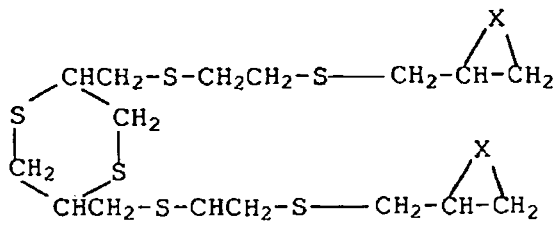

(XI) 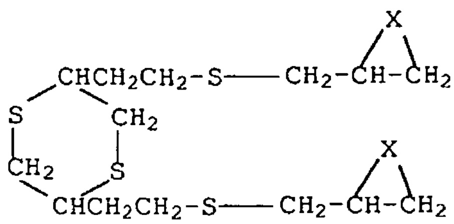

(XII) 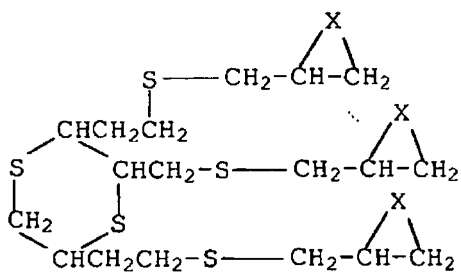

wherein X can be as defined above.

In alternate non-limiting embodiments, the sulfur-containing polyureaurethane of the present invention can have a equivalent ratio of ($-NH_2$ + -NH- + -OH + SH) to (NCO + NCS) of at least 0.4:1, or at least 0.8:1, or 1.0:1, or 2:0:1.0 or less.

In alternate non-limiting embodiments, the episulfide-containing material can be present in an amount such that the ratio of episulfide to (NCO + OH + SH) can be at least 0.01:1, or 1:1, or at least 1.3:1.0, or 4.0:1.0 or less, or 6.0:1.0 or less.

In a non-limiting embodiment, the polyurethane prepolymer can be the reaction product of Desmodur W and

a poly(caprolactone) diol having the general formula XXXI:

$$\text{(XXXI)} \qquad HO\text{-}[\text{-}(CH_2)_5\text{-}C(O)\text{-}o]_t\text{-}(CH_2)_5\text{-}OH$$

where t is an integer from 1 to 10.

In a non-limiting embodiment, the polyether-containing polyol can comprise block polymers including blocks of ethylene oxide-propylene oxide and/or ethylene oxide-butylene oxide. In a non-limiting embodiment, the polyether-containing polyol can comprise a block polymer of the following chemical formula:

$$H\text{-}(O\text{-}CRRCRR\text{-}Y_n)_a\text{-}(CRRCRR\text{-}Y_n\text{-}O)_b\text{-}(CRRCRR\text{-}Y_n\text{-}O)_c\text{-}H \qquad \text{(XXXI')}$$

wherein R can represent hydrogen or $C_1$-$C_6$ alkyl; Y can represent $CH_2$; n can be an integer from 0 to 6; a, b, and c can each be an integer from 0 to 300, wherein a, b and c are chosen such that the number average molecular weight of the polyol does not exceed 32,000 grams/mol.

In a non-limiting embodiment, the polyurethane prepolymer can be reacted with an episulfide-containing material of the structural formula XXXII:

(XXXII) 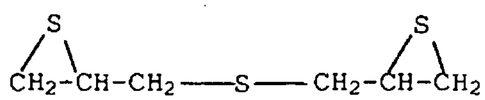

In alternate non-limiting embodiments, various known additives can be incorporated into the sulfur-containing polyureaurethane of the present invention. Such additives can include but are not limited to light stabilizers, heat stabilizers, antioxidants, ultraviolet light absorbers, mold release agents, static (non-photochromic) dyes, pigments and flexibilizing additives, such as but not limited to alkoxylated phenol benzoates and poly(alkylene glycol) dibenzoates. Non-limiting examples of anti-yellowing additives can include 3-methyl-2-butenol, organo pyrocarbonates and triphenyl phosphite (CAS registry no. 101-02-0). Such additives can be present in an amount such that the additive constitutes less than 10 percent by weight, or less than 5 percent by weight, or less than 3 percent by weight, based on the total weight of the prepolymer. In alternate non-limiting embodiments, the aforementioned optional additives can be mixed with the polycyanate. In a further embodiment, the optional additives can be mixed with hydrogen-containing material.

In a non-limiting embodiment, the resulting sulfur-containing polyureaurethane of the present invention when at least partially cured can be solid, and essentially transparent such that it is suitable for optical or ophthalmic applications. In alternate non-limiting embodiments, the sulfur-containing polyureaurethane can have a refractive index of at least 1.57, or at least 1.58, or at least 1.60, or at least 1.62. In further alternate non-limiting embodiments, the sulfur-containing polyureaurethane can have an Abbe number of at least 32, or at least 35, or at least 38, or at least 39, or at least 40, or at least 44.

In a non-limiting embodiment, the sulfur-containing polyureaurethane when polymerized and at least partially cured can demonstrate good impact resistance/strength. Impact resistance can be measured using a variety of conventional methods known to one skilled in the art. In a non-limiting embodiment, the impact resistance is measured using the Impact Energy Test which consists of testing a flat sheet of polymerizate having a thickness of 3mm, by dropping various balls of increasing weight from a distance of 50 inches (1.25 meters) onto the center of the sheet. If the sheet is determined to have failed the test when the sheet fractures. As used herein, the term "fracture" refers to a crack through the entire thickness of the sheet into two or more separate pieces, or detachment of one or more pieces from the backside of the sheet (i.e., the side of the sheet opposite the side of impact). In this embodiment, using the Impact Energy Test as described herein, the impact strength can be at least 2.0 joules, or at least 4.95 joules.

Further, the sulfur-containing polyureaurethane of the present invention when at least partially cured can have low density. In a non-limiting embodiment, the density can be from greater than 1.0 to less than 1.25 grams/$cm^3$, or from greater than 1.0 to less than 1.3 grams/$cm^3$. In a non-limiting embodiment, the density is measured using a DensiTECH instrument manufactured by Tech Pro, Incorporated. In a further non-limiting embodiment, the density is measured in accordance with ASTM D297.

Solid articles that can be prepared using the sulfur-containing polyureaurethane of the present invention include but are not limited to optical lenses, such as plano and ophthalmic lenses, sun lenses, windows, automotive transparencies, such as windshields, sidelights and backlights, and aircraft transparencies.

In a non-limiting embodiment, the sulfur-containing polyureaurethane polymerizate of the present invention can be used to prepare photochromic articles, such as lenses. In a further embodiment, the polymerizate can be transparent to that portion of the electromagnetic spectrum which activates the photochromic substance(s), i.e., that wavelength of ultraviolet (UV) light that produces the colored or open form of the photochromic substance and that portion of the visible

spectrum that includes the absorption maximum wavelength of the photochromic substance in its UV activated form, i.e., the open form.

A wide variety of photochromic substances can be used in the present invention. In a non-limiting embodiment, organic photochromic compounds or substances can be used. In alternate non-limiting embodiments, the photochromic substance can be incorporated, e.g., dissolved; dispersed or diffused into the polymerizate, or applied as a coating thereto.

In a non-limiting embodiment, the organic photochromic substance can have an activated absorption maximum within the visible range of greater than 590 nanometers. In a further non-limiting embodiment, the activated absorption maximum within the visible range can be between greater than 590 to 700 nanometers. These materials can exhibit a blue, bluish-green, or bluish-purple color when exposed to ultraviolet light in an appropriate solvent or matrix. Non-limiting examples of such substances that are useful in the present invention include but are not limited to spiro(indoline)naphthoxazines and spiro(indoline)benzoxazines. These and other suitable photochromic substances are described in U.S. Patents: 3,562,172; 3,578,602; 4,215,010; 4,342,668; 5,405,958; 4,637,698; 4,931,219; 4,816,584; 4,880,667; 4,818,096.

In another non-limiting embodiment, the organic photochromic substances can have at least one absorption maximum within the visible range of between 400 and less than 500 nanometers. In a further non-limiting embodiment, the substance can have two absorption maxima within this visible range. These materials can exhibit a yellow-orange color when exposed to ultraviolet light in an appropriate solvent or matrix. Non-limiting examples of such materials can include certain chromenes, such as but not limited to benzopyrans and naphthopyrans. Many of such chromenes are described in U.S. Patents 3,567,605; 4,826,977; 5,066,818; 4,826,977; 5,066,818; 5,466,398; 5,384,077; 5,238,931; and 5,274,132.

In another non-limiting embodiment, the photochromic substance can have an absorption maximum within the visible range of between 400 to 500 nanometers and an absorption maximum within the visible range of between 500 to 700 nanometers. These materials can exhibit color(s) ranging from yellow/brown to purple/gray when exposed to ultraviolet light in an appropriate solvent or matrix. Non-limiting examples of these substances can include certain benzopyran compounds having substituents at the 2-position of the pyran ring and a substituted or unsubstituted heterocyclic ring, such as a benzothieno or benzofurano ring fused to the benzene portion of the benzopyran. Further non-limiting examples of such materials are disclosed in U.S. Patent No. 5,429,774.

In a non-limiting embodiment, the photochromic substance for use in the present invention can include photochromic organo-metal dithizonates, such as but not limited to (arylazo)-thioformic arylhydrazidates, such as but not limited to mercury dithizonates which are described, for example, in U.S. Patent 3,361,706. Fulgides and fulgimides, such as but not limited to 3-furyl and 3-thienyl fulgides and fulgimides which are described in U.S. Patent 4,931,220 at column 20, line 5 through column 21, line 38, can be used in the present invention.

In alternate non-limiting embodiments, the photochromic articles of the present invention can include one photochromic substance or a mixture of more than one photochromic substances. In further alternate non-limiting embodiment, various mixtures of photochromic substances can be used to attain activated colors such as a near neutral gray or brown.

The amount of photochromic substance employed can vary. In alternate non-limiting embodiments, the amount of photochromic substance and the ratio of substances (for example, when mixtures are used) can be such that the polymerizate to which the substance is applied or in which it is incorporated exhibits a desired resultant color, e.g., a substantially neutral color such as shades of gray or brown when activated with unfiltered sunlight, i.e., as near a neutral color as possible given the colors of the activated photochromic substances. In a non-limiting embodiment, the amount of photochromic substance used can depend upon the intensity of the color of the activated species and the ultimate color desired.

In alternate non-limiting embodiments, the photochromic substance can be applied to or incorporated into the polymerizate by various methods known in the art. In a non-limiting embodiment, the photochromic substance can be dissolved or dispersed within the polymerizate. In a further non-limiting embodiment, the photochromic substance can be imbibed into the polymerizate by methods known in the art. The term "imbibition" or "imbibe" includes permeation of the photochromic substance alone into the polymerizate, solvent assisted transfer absorption of the photochromic substance into a porous polymer, vapor phase transfer, and other such transfer mechanisms. In a non-limiting embodiment, the imbibing method can include coating the photochromic article with the photochromic substance; heating the surface of the photochromic article; and removing the residual coating from the surface of the photochromic article. In alternate non-limiting embodiments, the imbibtion process can include immersing the polymerizate in a hot solution of the photochromic substance or by thermal transfer.

In alternate non-limiting embodiments, the photochromic substance can be a separate layer between adjacent layers of the polymerizate, e.g., as a part of a polymer film; or the photochromic substance can be applied as a coating or as part of a coating placed on the surface of the polymerizate.

The amount of photochromic substance or composition containing the same applied to or incorporated into the polymerizate can vary. In a non-limiting embodiment, the amount can be such that a photochromic effect discernible to

the naked eye upon activation is produced. Such an amount can be described in general as a photochromic amount. In alternate non-limiting embodiments, the amount used can depend upon the intensity of color desired upon irradiation thereof and the method used to incorporate or apply the photochromic substance. In general, the more photochromic substance applied or incorporated, the greater the color intensity. In a non-limiting embodiment, the amount of photochromic substance incorporated into or applied onto a photochromic optical polymerizate can be from 0.15 to 0.35 milligrams per square centimeter of surface to which the photochromic substance is incorporated or applied.

In another embodiment, the photochromic substance can be added to the sulfur-containing polyureaurethane prior to polymerizing and/or cast curing the material. In this embodiment, the photochromic substance used can be chosen such that it is resistant to potentially adverse interactions with, for example, the isocyanate, isothiocyante and amine groups present. Such adverse interactions can result in deactivation of the photochromic substance, for example, by trapping them in either an open or closed form.

Further non-limiting examples of suitable photochromic substances for use in the present invention can include photochromic pigments and organic photochromic substances encapsulated in metal oxides such as those disclosed in U.S. Patents 4,166,043 and 4,367,170; organic photochromic substances encapsulated in an organic polymerizate such as those disclosed in U.S. Patent 4,931,220.

## EXAMPLES

In the following examples, unless otherwise stated, the 1H NMR and 13C NMR were measured on a Varian Unity Plus (200 MHz) machine; the Mass Spectra were measured on a Mariner Bio Systems apparatus; the refractive index and Abbe number were measured on a multiple wavelength Abbe Refractometer Model DR-M2 manufactured by ATAGO Co., Ltd.; the refractive index and Abbe number of liquids were measured in accordance with ASTM-D1218; the refractive index and Abbe number of solids was measured in accordance with ASTM-D542; the density of solids was measured in accordance with ASTM-D792; and the viscosity was measured using a Brookfield CAP 2000+ Viscometer.

## REFERENCE EXAMPLE 1 - Synthesis of Polythioether (PTE) Dithiol (1)

NaOH (44.15 g, 1.01 mol) was dissolved in 350 ml of $H_2O$. The solution was allowed to cool to room temperature and 500 ml of toluene were added, followed by the addition of dimercaptoethylsulfide (135 ml, 159.70 g, 1.04 mol). The reaction mixture was heated to a temperature of 40°C, stirred and then cooled to room temperature. 1, 1 -Dichloroacetone (DCA) (50 ml, 66.35 g, 0.52 mol) was dissolved in 250 ml of toluene and then added drop-wise to the reaction mixture while the temperature was maintained at from 20-25°C. Following the drop-wise addition, the reaction mixture was stirred for an additional 20 hours at room temperature. The organic phase was than separated, washed with 2x100 ml of $H_2O$, 1x100 ml of brine and dried over anhydrous $MgSO_4$. The drying agent was filtered off and the toluene was evaporated using a Buchi Rotaevaporator. The hazy residue was filtered through Celite to provide 182 g (96% yield) of PTE Dithiol (1) as colorless clear oily liquid.

A Mass Spectra was conducted on a product sample using a Mariner Bio Systems apparatus. The results were as follows: ESI-MS: 385 (M + Na). Therefore, the molecular weight was 362.

A NMR was conducted on a product sample using a Varian Unity Plus machine. The results were as follows: $^1H$ NMR ($CDCl_3$, 200 MHz): 4.56 (s, 1H), 2.75 (m, 16 H), 2.38 (s, 3H), 1.75 (m, 1.5H)).

The SH groups within the product were determined using the following procedure. A sample size (0.1 mg) of the product was combined with 50 mL of tetrahydrofuran (THF)/propylene glycol (80/20) and stirred at room temperature until the sample was substantially dissolved. While stirring, 25.0 mL of 0.1 N iodine solution (which was commercially obtained from Aldrich 31, 8898-1) was added to the mixture and then allowed to react for a time period of from 5 to 10 minutes. To this mixture was added 2.0 mL concentrated HCl. The mixture was then titrated potentiometrically with 0.1 N sodium thiosulfate in the millivolt (mV) mode. A blank value was initially obtained by titrating 25.0 mL of iodine (including 1 mL of concentrated hydrochloric acid) with sodium thiosulfate in the same manner as conducted with the product sample.

$$\%SH = \frac{\text{mLsBlank} - \text{mLSSample})(\text{Normality Na2S2O3})(3.307)}{\text{sample weight, g}}$$

The following results were obtained: 13.4% SH

The refractive index (e-line) and the Abbe number were measured using a multiple wavelength Abbe Refractometer Model No. DR-M2, manufactured by ATAGO Co., Limited, in accordance with ASTM 542-00. The refractive index was 1.618 (20°C) and the Abbe number was 35.

The product sample was acetylated by the following procedure: PTE Dithiol (1) (100 mg, 0.28 mmol) was dissolved in 2 ml of dichloromethane at room temperature. Acetic anhydride (0.058 ml, 0.6 mmol) was added to the reaction mixture, and triethylamine (0.09 ml, 0.67 mmol) and dimethylaminopyridine (1 drop) were then added. The mixture was maintained at room temperature for 2 hours. The mixture was then diluted with 20 ml of ethyl ether, washed with aqueous $NaHCO_3$ and dried over $MgSO_4$. The drying agent was filtered off, the volatiles were evaporated off under vacuum and the oily residue was purified by silica gel flash chromatographed (hexane / ethyl acetate 8:2 v/v) to provide 103 mg (83% yield) of diacetylated product.

$^1$H NMR (CDCl3, 200 MHz): 4.65 (s, 1H), 3.12-3.02 (m, 4H), 2.75-2.65 (m, 4H), 2.95-2.78 (m, 8 H), 2.38 (s, 3H), 2.35 (s, 6H).

ESI-MS: 385 (M + Na).

$^1$H NMR ($CDCl_3$, 200 MHz), 4.56 (s, 1H), 2.75 (m, 16 H), 2.38 (s, 3H), 1.75 (m, 1.5H)).

<u>REFERENCE EXAMPLE 2 - Synthesis of PTE Dithiol (2)</u>

NaOH (23.4 g, 0.58 mol) was dissolved in 54 ml of $H_2O$. The solution was cooled down to room temperature and DMDS (30.8 g, 0.20 mol) was added. Upon stirring the mixture, dichloroacetone (19.0 g, 0.15 mol) was added dropwise while the temperature was maintained at from 20-25°C. After the addition of dichloroacetone was completed, the mixture was stirred for an additional 2 hours at room temperature. The mixture was acidified with 10% HCl to a pH<9, and 100 ml of dichloromethane were then added, and the mixture was stirred. Following phase separation, the organic phase was washed with 100 ml of $H_2O$, and dried over anhydrous $MgSO_4$. The drying agent was filtered off and the solvent was evaporated using a Buchi Rotaevaporator, which provided 35 g (90%) of viscous, transparent liquid having a viscosity (73°C) of 38 mPa·s (cP) ; refractive index (e-line) of 1.622 (20°C), Abbe number of 36, SH group analysis of 8.10%.

<u>REFERENCE EXAMPLE 3 - Synthesis of PTE Dithiol 3</u>

NaOH (32.0 g, 0.80 mol) was dissolved in 250 ml of $H_2O$. The solution was cooled to room temperature and 240 ml of toluene were added followed by the addition of DMDS (77.00 g, 0.50 mol). The mixture was heated to a temperature of 40°C, stirred and then cooled down under nitrogen flow until room temperature was reached. DCA (50.8 g, 0.40 mol) was dissolved in 70 ml of toluene and added dropwise to the mixture with stirring, while the temperature was maintained from 20-25°C. After the addition was completed, the mixture was stirred for additional 16 hours at room temperature. The organic phase was separated, washed with 2x100 ml of $H_2O$, 1x100 ml of brine and dried over anhydrous $MgSO_4$. The drying agent was filtered off and toluene was evaporated using a rotaevaporator to give 89 g (90%) of viscous, transparent liquid: viscosity (73°C): 58 mPa·s (cP); refractive index (e-line): 1.622 (20°C), Abbe number 36; SH group analysis: 3.54%.

<u>REFERENCE EXAMPLE 4 - Synthesis of PTE Dithiol 4</u>

NaOH (96.0 g, 2.40 mol) was dissolved in 160 ml of $H_2O$ and the solution was cooled to room temperature. DMDS (215.6 g, 1.40 mol), 1,1-dichloroethane (DCE) (240.0 g, 2.40 mol) and tetrabutylphosphonium bromide (8.14 g, 1 mol. %) were mixed and added to the above mixture dropwise under nitrogen flow and vigorous stirring while the temperature was kept at 20-25°C. After the addition was completed the mixture was stirred for additional 15 hours at room temperature. The aqueous layer was acidified and extracted to give 103.0 g unreacted DMDS. The organic phase was washed with 2x100 ml of $H_2O$, 1x100 ml of brine and dried over anhydrous $MgSO_4$. Drying agent was filtered off and the excess of DCE was evaporated on rotaevaporator to give 78 g (32%) transparent liquid: viscosity (73°C) : 15 mPa·s (cP); refractive index (e-line) : 1.625 (20°C), Abbe number 36; SH group analysis 15.74%.

<u>REFERENCE EXAMPLE 5 - Synthesis of PTE Dithiol 5</u>

NaOH (96.0 g, 2.40 mol) was dissolved in 140 ml of $H_2O$ and the solution was cooled to 10°C and charged in a three necked flask equipped with mechanical stirrer and inlet and outlet for Nitrogen. Then DMDS (215.6 g, 1.40 mol) was charged and the temperature was kept at 10°C. To this mixture was added dropwise the solution of tetrabutylphosphonium bromide (8.14 g, 1mol. %) in DCE (120 g, 1.2 mol) under Nitrogen flow and under vigorous stirring. After the addition was completed the mixture was stirred for additional 60 hours at room temperature. Then 300 ml of $H_2O$ and 50 ml of DCE were added, the mixture was shaken well and after phase separation, 200 ml toluene were added to the organic layer; then it was washed with 150 ml $H_2O$, 50 ml 5% HCl and 2x100 ml $H_2O$ and dried over anhydrous $MgSO_4$. Drying agent was filtered off and the solvent was evaporated on rotaevaporator to give 80 g (32%) of viscous, transparent liquid: viscosity (73°C): 56 mPa·s (cP); refractive index (e-line) : 1.635 (20°C), Abbe number 36; SH group analysis: 7.95%.

REFERENCE EXAMPLE 6 - Synthesis of Polythiourethane Prepolymer (PTUPP) 1

Desmodur W (62.9 g, 0.24 mol) and PTE Dithiol [1] (39.4 g, 0.08 mol) were mixed and degassed under vacuum for 2.5 hours at room temperature. Dibutyltin dilaurate (0.01 % by weight) was then added and the mixture was flushed with nitrogen and heated for 32 hours at 86°C. SH group analysis showed complete consumption of SH groups. The heating was stopped. The resulting viscous mixture had a viscosity (73°C) 600 mPa·s (cP) as measured on a Brookfield CAP 2000+ Viscometer, refractive index (e-line): 1.562 (20°C), Abbe number 43; NCO groups 13.2 % (calculated 13.1%). The NCO was determined according to the procedure described in Example 1 herein.

REFERENCE EXAMPLE 7 - Synthesis of PTUPP 2

Desmodur W (19.7 g, 0.075 mol) and PTE Dithiol [2] (20.0g, 0.025 mol) were mixed and degassed under vacuum for 2.5 hours at room temperature. Dibutyltin dichloride (0.01 weight percent) was then added to the mixture, and the mixture was flushed with nitrogen and heated for 18 hours at a temperature of 86°C. SH group analysis showed complete consumption of SH groups. The heating was stopped. The resulting viscous mixture had viscosity (at a temperature of 73°C) of 510 mPa·s (cP) as measured by a Brookfield CAP 2000+ Viscometer, refractive index (e-line): 1.574 (20°C), Abbe number 42; NCO groups 10.5 % (calculated 10.6%).

REFERENCE EXAMPLE 8 - Synthesis of PTUPP 3

Desmodur W (31.0 g, 0.118 mol) and PTE Dithiol [3] (73.7 g, 0.039 mol) were mixed and degassed under vacuum for 2.5 hours at room temperature. Dibutyltin dichloride was then added (0.01 weight percent) to the mixture, and the mixture was flushed with nitrogen and heated for 37 hours at a temperature of 64°C. SH group analysis showed complete consumption of SH groups. The heating was stopped. The resulting viscous mixture had viscosity (at a temperature of 73°C) of 415 mPa·s (cP) as measured using a Brookfield CAP 2000+ Viscometer, refractive index (e-line): 1.596 (20°C), Abbe number 39; NCO groups 6.6 % (calculated 6.3%).

EXAMPLE 9 - Chain Extension of Polythiourethane Prepolymer with Aromatic Amine

PTUPP [1] (30 g) was degassed under vacuum at a temperature of 70°C for 2 hours. DETDA (7.11 g) and PTE Dithiol [1] (1.0 g) were mixed and degassed under vacuum at a temperature of 70°C for 2 hours. The two mixtures were then mixed together at the same temperature and charged between preheated glass plates mold. The material was cured in a preheated oven at a temperature of 130°C for 5 hours. The cured material was transparent and had a refractive index (e-line): 1.585 (20°C), Abbe number 39 and density 1.174 g/cm$^3$. The density was determined in accordance with ASTM D792.

EXAMPLE 10

PTUPP 2 (25 g) was degassed under vacuum at 65oC for 3 hours. DETDA (3.88 g) and PTE Dithiol 1 (3.83 g) were mixed and degassed under vacuum at 65°C for 2 hours. Then the two mixtures were mixed at the same temperature and charged between preheated glass plates mold. The material was cured in a preheated oven at 130°C for 10 hours. The cured material is transparent and has refractive index (e-line): 1.599 (20°C), Abbe number 39 and density 1.202 g/cm$^3$.

EXAMPLE 11

PTUPP 3 (40 g) was degassed under vacuum at 65°C for 2 hours. DETDA (3.89 g) and PTE Dithiol 1 (3.84 g) were mixed and degassed under vacuum at 65°C for 2 hours. Then the two mixtures were mixed at the same temperature and charged between preheated glass plates mold. The material was cured in a preheated oven at 130°C for 10 hours. The cured material is transparent and has refractive index (e-line): 1.609 (20°C), Abbe number 39 and density 1.195 g/cm$^3$.

REFERENCE EXAMPLE 12 - Synthesis of 2-Methyl-2-Dichloromethyl-1,3-Dithiolane

In a three-necked flask equipped with a magnetic stirrer and having a nitrogen blanket at the inlet and outlet, were added 13.27 grams (0.104 mol) of 1,1-dichloroacetone, 11.23 grams (0.119 mol) of 1,2-ethanedithiol, 20 grams of $MgSO_4$ anhydrous, and 5 grams of Montmorilonite K-10 (commercially obtained from Aldrich, USA) in 200 ml toluene. The mixture was stirred for 24 hours at room temperature. The insoluble product was filtered off and the toluene was evaporated off under vacuum to give 17.2 grams (80% yield) of a crude 2-methyl-2-dichloromethyl-1,3-dithiolane.

The crude product was distilled within a temperature range of from 102 to 112°C at 1.6 kPa (12 mm Hg). $^{1}H$ NMR and $^{13}C$ NMR of the distilled product was measured using a Varian Unity Plus (200 MHz) machine. The results were as follows: $^{1}H$ NMR (CDC13, 200 MHz): 5.93 (s, 1H); 3.34 (s, 4H); 2.02 (s, 3H); $^{13}C$ NMR (CDCl3, 50MHz): 80.57; 40.98; 25.67.

REFERENCE EXAMPLE 13 - Synthesis of PTE Dithiol 6 (DMDS/VCH, 1:2 mole ratio)

Charged into a 1-liter 4-necked flask equipped with a mechanical stirrer, thermometer and two gas passing adapters (one for inlet and one for outlet), 2-dimercaptoethyl sulfide (DMDS) (888.53g, 5.758 moles). The flask was flushed with dry nitrogen and 4-vinyl-1-cyclohexene (VCH) (311.97g, 2.879 moles) was added with stirring during a time period of 2hr, 15 min. The reaction temperature increased from room temperature to 62°C after 1 hr of addition. Following addition of the vinylcyclohexene, the reaction temperature was 37°C. The reaction mixture was then heated to a temperature of 60°C, and five 0.25g-portions of free radical initiator Vazo-52 (2,2'-azobis(2,4-dimethylpentanenitrile) obtained from DuPont), were added. Each portion was added after interval of one hour. The reaction mixture was evacuated at 60°C/533-667 kPa (4-5mm Hg) for one hour to give 1.2 kg (yield: 100%) of a colorless liquid , with the following properties:

Viscosity 300 mPa·s (cps) @ 25°C (Brookfield CAP 2000+, spindle #3, 500 rpm); refractive index (e-line) = 1.597 (20°C); Abbe Number = 39; SH groups content 16.7%.

REFERENCE EXAMPLE 14 - Synthesis of PTE Dithiol 7 (DMDS/VCH, 5:4 mole ratio)

In a glass jar with magnetic stirrer were mixed 21.6.8 grams (0.20 mole) of 4-vinyl-1-cyclohexene (VCH) from Aldrich and 38.6 grams (0.25 mole) of 2-mercaptoethyl sulfide (DMDS) from Nisso Maruzen. The mixture had a temperature of 60°C due to the exothermicity of the reaction. The mixture was placed in an oil bath at a temperature of 47°C and stirred under a nitrogen flow for 40 hours. The mixture was then cooled to room temperature. A colorless, viscous oligomeric product was obtained, with the following properties:

Viscosity 10860 mPa·s (cps) @ 25 °C (Brookfield CAP 2000+, spindle #3, 50 rpm); refractive index (e-line) = 1.604 (20°C); Abbe Number = 41; SH groups content 5.1%.

REFERENCE EXAMPLE 15 - Synthesis of Star Polymer (SP)

In a glass-lined reactor of 7500 lb capacity, were added 1,8-dimercapto-3,6-dioxaooctane (DMDO) (3907.541b, 21.43moles), ethyl formate (705.53 lb, 9.53 moles), and anhydrous zinc chloride (90.45 1b, 0.66 mole). The mixture was stirred at a temperature of 85°C for 20 hours, then cooled to a temperature of 52°C. Added to the mixture was 96.48 lb of a 33% solution of 1,4-diazabicyclo[2.2.2]octane (DABCO) (0.28mole) for one hour. The mixture was then cooled to a temperature of 49°C, and filtered through a 200-micron filter bag to provide a liquid polythioether with the following properties:

Viscosity, 320 mPa·s (cps) @ 25 °C (Brookfield CAP 2000+, spindle #1, 1000rpm); $n_D^{20}$ = 1.553; Abbe Number = 42; SH groups content 11.8% (thiol equivalent weight.: 280).

REFERENCE EXAMPLE 16 - Synthesis of PTUPP 4

4,4-dicyclohexylmethane diisocyanate (Desmodur W) from Bayer (20.96g, 0.08 mole), Isophorone diisocyanate (IPDI) from Bayer (35.52g, 0.16 mole) and PTE Dithiol 6 (32.0 g, 0.08 mole) were mixed and degassed under vacuum for 2.5 hours at room temperature. Dibutyltin dilaurate (0.01%) obtained from Aldrich was then added to the mixture and the mixture was flushed with Nitrogen and heated for 16 hours at a temperature of 90°C. SH group analysis showed complete consumption of SH groups. The heating was terminated. The resulting clear viscous mixture had viscosity (73°C) 1800 mPa·s (cP), refractive index (e-line): 1.555 (20°C), Abbe number 44; NCO groups 14.02 %.

EXAMPLE 17 - Chain extension of PTUPP 4

PTUPP 4 (30 g) was degassed under vacuum at a temperature of 60°C for two hours. DETDA (7.57 g) and PTE Dithiol 6 (2.02g) were mixed and degassed under vacuum at a temperature of 60°C for 2 hours. Then the two mixtures were mixed at the same temperature and charged between preheated glass plates mold. The material was cured in a preheated oven at a temperature of 130°C for five hours. The cured material was clear and had refractive index (e-line): 1.574 (20°C), Abbe number 40.

REFERENCE EXAMPLE 18 - Synthesis of PTUPP 5

4,4-dicyclohexylmethane diisocyanate (Desmodur W) from Bayer (99.00g, 0.378 mole), PTE Dithiol 6 (47.00g, 0.118 mole) and Star Polymer (Example 6) (4.06 g, 0.0085 mole) were mixed and degassed under vacuum for 2.5 hours at room temperature. Then Dibutyltin dilaurate (Aldrich) was added (0.01 %) and the mixture was flushed with Nitrogen and heated for 16 hours at 90°C. SH group analysis showed complete consumption of SH groups. The heating was stopped. The resulting clear viscous mixture had viscosity (73°C) 1820 mPa·s (cP), refractive index (e-line): 1.553 (20°C), Abbe number 46; NCO groups 13.65 %.

EXAMPLE 19 - Chain extension of PTUPP 5

PTUPP 5 (30 g) was degassed under vacuum at a temperature of 60°C for two hours. DETDA (6.94 g) and DMDS (1.13g) were mixed and degassed under vacuum at a temperature of 60°C for two hours. The two mixtures were then mixed at the same temperature and charged between preheated glass plates mold. The material was cured in a preheated oven at a temperature of 130°C for five hours. The cured material was clear and had refractive index (e-line): 1.575 (20°C), Abbe number 41.

**Claims**

1. A sulfur-containing polyureaurethane adapted to have a refractive index of at least 1.57 determined by ASTM D 542-00, an Abbe number of at least 32 determined by ASTM D 542-00, and a density of less than 1.3 grams/$cm^3$, when at least partially cured, that is prepared by the reaction of

   (a) a sulfur-containing polyurethane prepolymer being the reaction product of

   (aa1) a sulfur-containing polycyanate and
   (aa2) an active hydrogen-containing material or
   (ab1) a polyisocyanate and
   (ab2) a sulfur-containing active hydrogen material; and

   (b) an amine-containing curing agent,

   wherein said amine-containing curing agent is a mixture of a polyamine and at least one material chosen from a polythiol (pb) and polyol and wherein

   (i) the sulfur-containing polyurethane is the reaction product of a polyisocyanate (ab1) and a sulfur-containing active hydrogen material (ab2) which is a polythiol (pa) comprising at least one material represented by the following structural formulas:

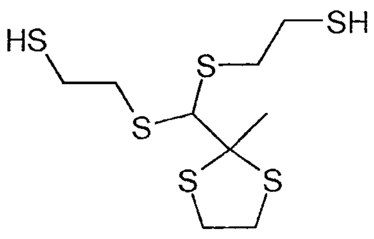

(IV'a)

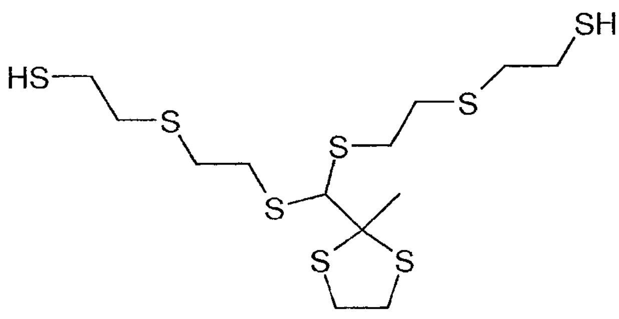

(IV'b)

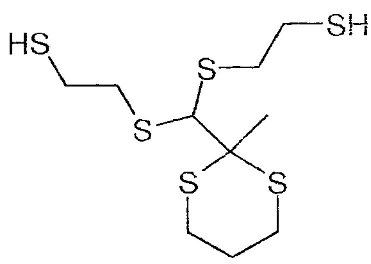

(IV'c)

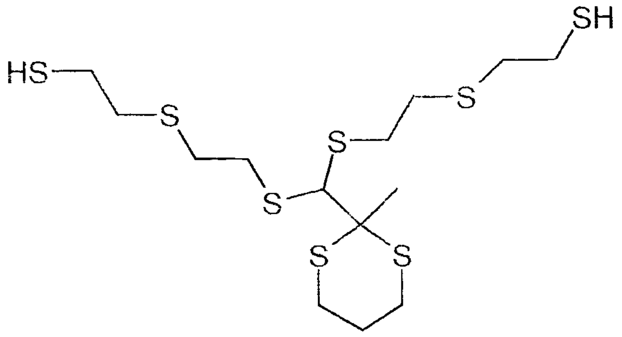

(IV'd)

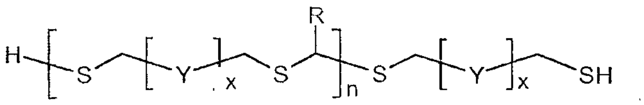

(IV'e)

wherein R can represent $CH_3$, $CH_3CO$, $C_1$ to $C_{10}$ alkyl, cycloalkyl, aryl alkyl, or alkyl-CO; Y can represent $C_1$ to $C_{10}$ alkyl, cycloalkyl, $C_6$ to $C_{14}$ aryl, $(CH_2)_p(S)_m(CH_2)_q$, $(CH_2)_p(Se)_m(CH_2)_q$, $(CH_2)_p(Te)_m(CH_2)_q$ wherein m can be an integer from 1 to 5 and, p and q can each be an integer from 1 to 10; n can be an integer from 1 to 30; and x can be an integer from 0 to 10,

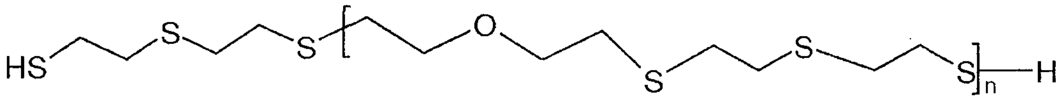

(IV'f)

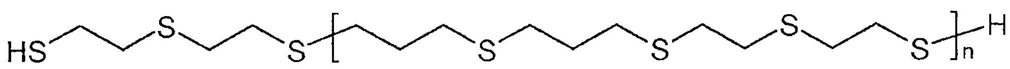

(IV'g)

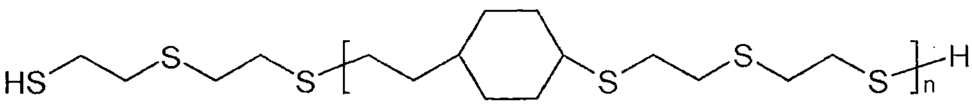

(IV'h)

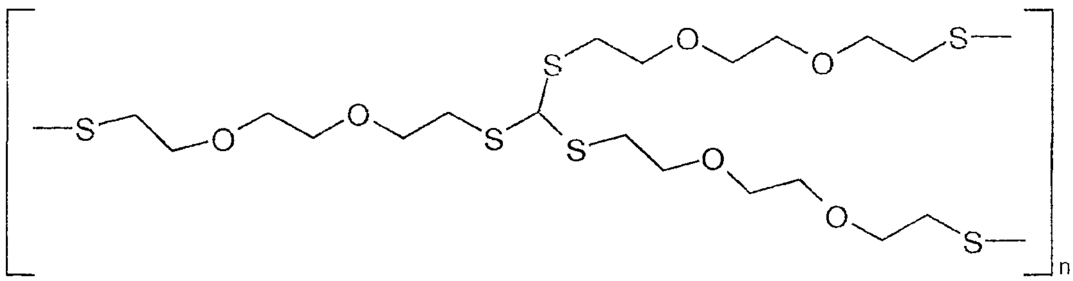

(IV'i)

wherein n can be an integer from 1 to 20, and/or
(ii) the polythiol (pb) comprises at least one material represented by the structural formulas (IV'a) to (IV'i) above.

**2.** The sulfur-containing polyureaurethane of claim 1 wherein the sulfur-containing polycyanate comprises a polyisothiocyanate or a mixture of a polyisothiocyanate and a polyisocyanate.

**3.** The sulfur-containing polyureaurethane of claim 1 wherein the active hydrogen-containing material comprises

a) a polyol free of sulfur;
b) a polythiol;
c) a mixture of a polyol free of sulfur and a polythiol; or
d) a hydroxyl functional polysulfide.

**4.** The sulfur-containing polyureaurethane of claim 3 wherein said hydroxyl functional polysulfide further comprises SH-functionality.

**5.** The sulfur-containing polyureaurethane of claim 3 wherein said polyol free of sulfur is chosen from polyester polyols, polycaprolactone polyols, polyether polyols, polycarbonate polyols, and mixtures thereof, preferably the polyol free of sulfur comprises a polyether polyol.

**6.** The sulfur-containing polyureaurethane of claim 1 wherein said active hydrogen-containing material has a number average molecular weight of from 200 grams/mol to 32,000 grams/mol, preferably 2,000 to 15,000 grams/mol as determined by GPC.

**7.** The sulfur-containing polyureaurethane of claim 3 wherein said polyol free of sulfur comprises a polyether polyol.

**8.** The sulfur-containing polyureaurethane of claim 7 wherein said polyether polyol is represented by the following structural formula:

$$H\text{-}(O\text{-}CRRCRR\text{-}Y_n)_a\text{-}(CRRCRR\text{-}Y_n\text{-}O)_b\text{-}(CRRCRR\text{-}Y_n\text{-}O)_c\text{-}H$$

wherein R can represent hydrogen or $C_1$-$C_6$ alkyl; Y can represent $CH_2$; n can be an integer from 0 to 6; a, b, and c can each be an integer from 0 to 300, wherein a, b and c are chosen such that the number average molecular weight of the polyol does not exceed 32,000 grams/mol as determined by GPC.

**9.** The sulfur-containing polyureaurethane of claim 1 wherein said sulfur-containing polycyanate and said active hydrogen-containing material are present in an amount such that the molar equivalent ratio of (NCO + NCS) to (SH + OH) or (SH + OH + NR), wherein R is hydrogen or alkyl, is less than 5.5 to 1.0.

**10.** The sulfur-containing polyureaurethane of claim 1 wherein the polyisocyanate is chosen from aliphatic polyisocyanates, cycloaliphatic polyisocyanates, aromatic polyisocyanates, and mixtures thereof.

**11.** The sulfur-containing polyureaurethane of claim 1 wherein said polyisocyanate is chosen from aliphatic diisocyanates, cycloaliphatic diisocyanates, aromatic diisocyanates, cyclic dimers and cyclic trimers thereof, and mixtures thereof.

**12.** The sulfur-containing polyureaurethane of claim 1 wherein said polyisocyanate is chosen from cyclohexylmethane diisocyanate and isomeric mixtures thereof; trans, trans isomer of 4,4'-methylenebis(cyclohexyl isocyanate); 3-isocyanato-methyl-3,5,5-trimethyl cyclohexyl-isocyanate; meta-tetramethylxylene diisocyanate (1,3-bis(1-isocyanato-1-methylethyl)-benzene); and mixtures of 3-isocyanato-methyl-3,5,5-trimethyl cyclohexyl-isocyanate and meta-tetramethylxylene diisocyanate (1,3-bis(1-isocyanato-1-methylethyl)-benzene).

**13.** The sulfur-containing polyureaurethane of claim 1 wherein the sulfur-containing active hydrogen material is a SH-containing material, preferably a polythiol.

**14.** The sulfur-containing polyureaurethane of claims 1 or 13 wherein said polythiol is chosen from aliphatic polythiols, cycloaliphatic polythiols, aromatic polythiols, polymeric polythiols, polythiols containing ether linkages, polythiols containing one or more sulfide linkages.

**15.** The sulfur-containing polyureaurethane of claim 13 wherein the SH-containing material comprises a mixture of polythiol and polyol free of sulfur.

**16.** The sulfur-containing polyureaurethane of claim 1 wherein the sulfur-containing active hydrogen material is a hydroxyl functional polysulfide, wherein said hydroxyl functional polysulfide preferably further comprises SH-functionality.

**17.** The sulfur-containing polyureaurethane of claim 1 wherein said polyamine has at least two functional groups independently chosen from primary amine ($-NH_2$), secondary amine (-NH-), and combinations thereof.

**18.** The sulfur-containing polyureaurethane of claim 17 wherein said polyamine is chosen from aliphatic polyamines, cycloaliphatic polyamines, aromatic polyamines, and mixtures thereof.

**19.** The sulfur-containing polyureaurethane of claim 17 wherein said polyamine is represented by the following structural formula and mixtures thereof:

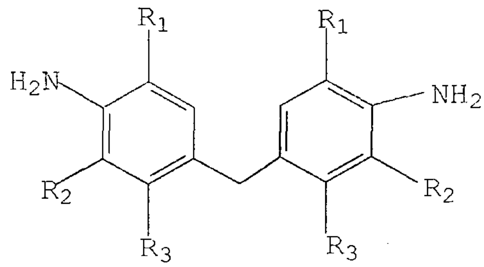

wherein $R_1$ and $R_2$ are each independently chosen from methyl, ethyl, propyl, and isopropyl groups, and $R_3$ is chosen from hydrogen and chlorine.

**20.** The sulfur-containing polyureaurethane of claim 17 wherein said polyamine is chosen from is 4,4'-methylenebis(3-chloro-2,6-diethylaniline); 2,4-diamino-3,5-diethyl-toluene; 2,6-diamino-3,5-diethyl-toluene; and mixtures of 2,4-diamino-3,5-diethyl-toluene and 2,6-diamino-3,5-diethyl-toluene.

**21.** A method of preparing a sulfur-containing polyureaurethane as defined by claim 1 comprising:

(a) reacting

(i) a sulfur-containing polycyanate and an active hydrogen-containing material or
(ii) a polyisocyanate with a sulfur-containing active hydrogen-containing material

to form a polyurethane prepolymer; and
(b) reacting said polyurethane prepolymer with an amine-containing curing agent,

wherein said amine-containing curing agent is a mixture of a polyamine and at least one material chosen from a polythiol and polyol.

**22.** The method of claim 21, wherein the sulfur-containing polycyanate, the polyisocyanate, the active hydrogen-containing material, the sulfur-containing active hydrogen-containing material and the amine-containing curing agent are defined as in any of claims 2 to 20.

**23.** An optical article comprising a sulfur-containing polyureaurethane as defined in any of claims 1 to 20.

**24.** The optical article of claim 23 being selected from an ophthalmic lens and a photochromic article.

**25.** The optical article of claim 24 being a photochromic article wherein it comprises an at least partially cured substrate, and at least a photochromic amount of a photochromic substance.

**26.** The photochromic article of claim 25 wherein said photochromic substance is at least partially imbibed into said substrate.

**27.** The photochromic article of claim 25 wherein said substrate is at least partially coated with a coating composition comprising at least a photochromic amount of a photochromic substance.

**28.** The photochromic article of claim 25 wherein said photochromic substance is chosen from spiro(indoline)naphthoxazines, spiro(indoline)benzoxazines, benzopyrans, naphthopyrans, organo-metal dithizonates, fulgides and fulgimides, and mixtures thereof.

**Patentansprüche**

**1.** Schwefelhaltiges Polyharnstoffurethan, das angepasst ist, um einen Brechungsindex von mindestens 1,57, bestimmt nach ASTM D542-00, eine Abbe-Zahl von mindestens 32, bestimmt nach ASTM D542-00, und eine Dichte von weniger als 1,3 g/$cm^3$, wenn es zumindest teilweise gehärtet ist, aufzuweisen, das hergestellt ist durch die Reaktion von:

(a) einem schwefelhaltigen Polyurethanpräpolymer, das das Reaktionsprodukt ist von

(aa1) einem schwefelhaltigen Polycyanat und
(aa2) einem aktiven Wasserstoff enthaltenden Material oder
(ab1) einem Polyisocyanat und
(ab2) einem schwefelhaltigen aktiven Wasserstoff enthaltenden Material und

(b) einem aminhaltigen Härtungsmittel,

wobei dieses aminhaltige Härtungsmittel eine Mischung aus einem Polyamin und mindestens einem Material ausgewählt aus einem Polythiol (pb) und einem Polyol ist und wobei

(i) das schwefelhaltige Polyurethan das Reaktionsprodukt eines Polyisocyanats (ab1) und eines schwefelhaltigen aktiven Wasserstoff enthaltenden Materials (ab2) ist, das ein Polythiol (pa) ist, das mindestens ein Material dargestellt durch die folgenden Strukturformeln enthält:

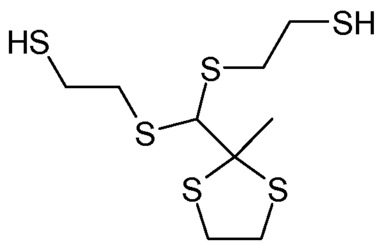

(IV'a)

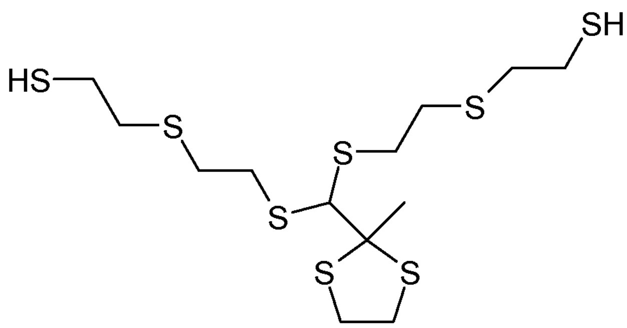

(IV'b)

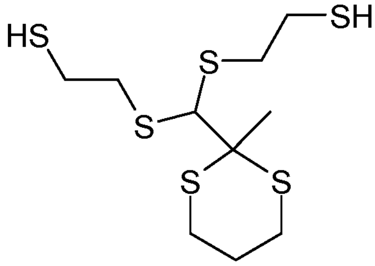

(IV'c)

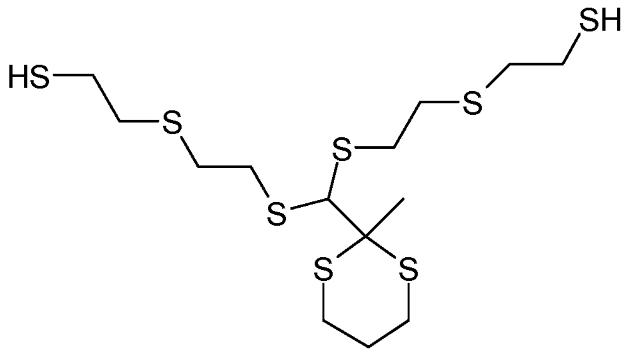

(IV'd)

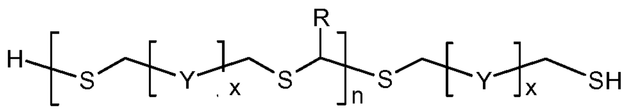

(IV'e)

worin R $CH_3$, $CH_3CO$, $C_1$- bis $C_{10}$-Alkyl, Cycloalkyl, Arylalkyl oder Alkyl-CO darstellen kann; Y $C_1$- bis $C_{10}$-Alkyl, Cycloalkyl, $C_6$- bis $C_{14}$-Aryl, $(CH_2)_p(S)_m(CH_2)_q$, $(CH_2)_p(Se)_m(CH_2)_q$, $(CH_2)_p(Te)_m(CH_2)_q$ darstellen kann, worin m eine ganze Zahl von 1 bis 5 sein kann und p und q jeweils eine ganze Zahl von 1 bis 10 sein können; n eine ganze Zahl von 1 bis 30 sein kann und x eine ganze Zahl von 0 bis 10 sein kann,

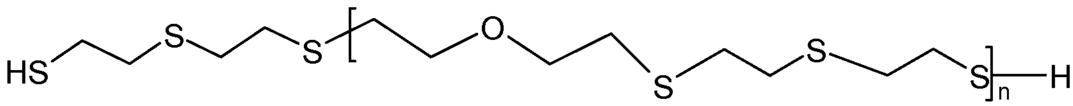

(IV'f)

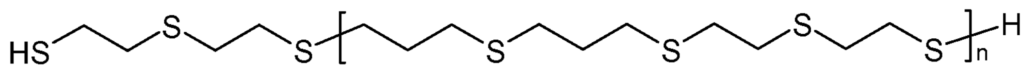

(IV'g)

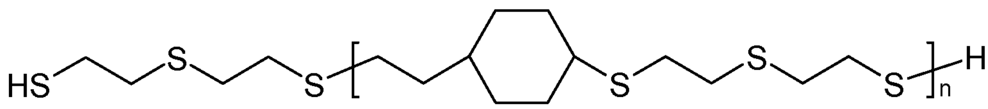

(IV'h)

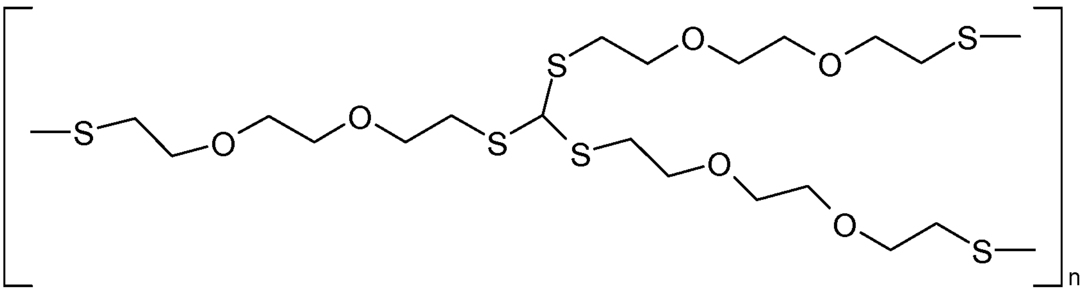

(IV'i)

worin n eine ganze Zahl von 1 bis 20 sein kann und/oder

(ii) das Polythiol (pb) mindestens ein Material dargestellt durch die obigen Strukturformeln (IV'a) bis (IV'i) enthält.

2. Schwefelhaltiges Polyharnstoffurethan nach Anspruch 1, wobei das schwefelhaltige Polycyanat ein Polyisothiocyanat oder eine Mischung eines Polyisothiocyanats und eines Polyisocyanats enthält.

3. Schwefelhaltiges Polyharnstoffurethan nach Anspruch 1, wobei das aktiven Wasserstoff enthaltende Material enthält:

a) ein schwefelfreies Polyol,
b) ein Polythiol,
c) eine Mischung eines schwefelfreien Polyols und eines Polythiols oder
d) ein hydroxylfunktionelles Polysulfid.

4. Schwefelhaltiges Polyharnstoffurethan nach Anspruch 3, worin dieses hydroxylfunktionelle Polysulfid ferner SH-Funktionalität aufweist.

5. Schwefelhaltiges Polyharnstoffurethan nach Anspruch 3, wobei dieses schwefelfreie Polyol aus Polyesterpolyolen, Polycaprolactonpolyolen, Polyetherpolyolen, Polycarbonatpolyolen und Mischungen derselben ausgewählt ist, vorzugsweise enthält das schwefelfreie Polyol ein Polyetherpolyol.

6. Schwefelhaltiges Polyharnstoffurethan nach Anspruch 1, wobei dieses aktiven Wasserstoff enthaltende Material ein zahlenmittleres Molekulargewicht von 200 g/mol bis 32.000 g/mol vorzugsweise 2.000 bis 15.000 g/mol, wie mittels GPC bestimmt, aufweist.

7. Schwefelhaltiges Polyharnstoffurethan nach Anspruch 3, wobei dieses schwefelfreie Polyol ein Polyetherpolyol enthält.

8. Schwefelhaltiges Polyharnstoffurethan nach Anspruch 7, wobei dieses Polyetherpolyol durch die folgende Strukturformel dargestellt wird:

$$H\text{-}(O\text{-}CRRCRR\text{-}Y_n)_a\text{-}(CRRCRR\text{-}Y_n\text{-}O)_b\text{-}(CRRCRR\text{-}Y_n\text{-}O)_c\text{-}H$$

worin R Wasserstoff oder $C_1$-$C_6$-Alkyl darstellen kann; Y $CH_2$ darstellen kann; n eine ganze Zahl von 0 bis 6 sein kann; a, b und c jeweils eine ganze Zahl von 0 bis 300 sein können, worin a, b und c ausgewählt sind, sodass das zahlenmittlere Molekulargewicht des Polyols 32.000 g/mol, wie mit Hilfe von GPC bestimmt, nicht übersteigt.

9. Schwefelhaltiges Polyharnstoffurethan nach Anspruch 1, wobei dieses schwefelhaltige Polycyanat und dieses aktiven Wasserstoff enthaltende Material in einer Menge vorhanden sind, sodass das molare Äquivalenzverhältnis von (NCO + NCS) zu (SH + OH) oder (SH + OH + NR), worin R Wasserstoff oder Alkyl ist, weniger als 5,5 bis 1,0 beträgt.

10. Schwefelhaltiges Polyharnstoffurethan nach Anspruch 1, wobei das Polyisocyanat aus aliphatischen Polyisocyanaten, cycloaliphatischen Polyisocyanaten, aromatischen Polyisocyanaten und Mischungen derselben ausgewählt ist.

**11.** Schwefelhaltiges Polyharnstoffurethan nach Anspruch 1, wobei dieses Polyisocyanat aus aliphatischen Diisocyanaten, cycloaliphatischen Diisocyanaten, aromatischen Diisocyanaten, cyclischen Dimeren und cyclischen Trimeren derselben und Mischungen daraus ausgewählt ist.

**12.** Schwefelhaltiges Polyharnstoffurethan nach Anspruch 1, wobei dieses Polyisocyanat aus Cyclohexylmethandiisocyanat und isomeren Mischungen daraus, trans,trans-Isomer von 4,4'-Methylenbis(cyclohexylisocyanat), 3-Isocyanatomethyl-3,5,5-trimethylcyclohexylisocyanat; meta-Tetramethylxylylendiisocyanat (1,3-Bis(1-isocyanato-1-methylethyl)benzol) und Mischungen von 3-Isocyanatomethyl-3,5,5-trimethylcyclohexylisocyanat und meta-Tetramethylxylylendiisocyanat (1,3-Bis(1-isocyanato-1-methylethyl)benzol) ausgewählt ist.

**13.** Schwefelhaltiges Polyharnstoffurethan nach Anspruch 1, wobei das schwefelhaltige aktiven Wasserstoff enthaltende Material ein SH-haltiges Material, vorzugsweise ein Polythiol ist.

**14.** Schwefelhaltiges Polyharnstoffurethan nach Ansprüchen 1 oder 13, wobei dieses Polythiol ausgewählt ist aus aliphatischen Polythiolen, cycloaliphatischen Polythiolen, aromatischen Polythiolen, polymeren Polythiolen, Polythiolen, die Etherverknüpfungen enthalten, Polythiolen, die eine oder mehrere Sulfidverknüpfungen enthalten.

**15.** Schwefelhaltiges Polyharnstoffurethan, nach Anspruch 13, wobei das SH-haltige Material eine Mischung aus Polythiol und schwefelfreiem Polyol enthält.

**16.** Schwefelhaltiges Polyharnstoffurethan nach Anspruch 1, wobei das schwefelhaltige aktiven Wasserstoff enthaltende Material ein hydroxylfunktionelles Polysulfid ist, wobei dieses hydroxylfunktionelle Polysulfid vorzugsweise ferner SH-Funktionalität aufweist.

**17.** Schwefelhaltiges Polyharnstoffurethan nach Anspruch 1, wobei dieses Polyamin mindestens zwei funktionelle Gruppen aufweist, die unabhängig voneinander ausgewählt sind aus primärem Amin ($-NH_2$), sekundärem Amin (-NH-) und Kombinationen daraus.

**18.** Schwefelhaltiges Polyharnstoffurethan nach Anspruch 17, wobei dieses Polyamin aus aliphatischen Polyaminen, cycloaliphatischen Polyaminen, aromatischen Polyaminen und Mischungen daraus ausgewählt ist.

**19.** Schwefelhaltiges Polyharnstoffurethan nach Anspruch 17, wobei dieses Polyamin durch die folgende Strukturformel und Mischungen daraus dargestellt wird:

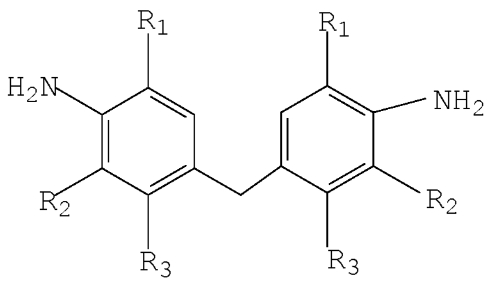

worin $R_1$ und $R_2$ jeweils unabhängig voneinander ausgewählt sind aus Methyl-, Ethyl-, Propyl- und Isopropylgruppen und $R_3$ aus Wasserstoff und Chlor ausgewählt ist.

**20.** Schwefelhaltiges Polyharnstoffurethan nach Anspruch 17, wobei dieses Polyamin aus 4,4'-Methylenbis(3-chlor-2,6-diethylanilin), 2,4-Diamino-3,5-diethyltoluol, 2,6-Diamino-3,5-diethyltoluol und Mischungen von 2,4-Diamino-3,5-diethyltoluol und 2,6-Diamino-3,5-diethyltoluol ausgewählt ist.

**21.** Verfahren zur Herstellung eines schwefelhaltigen Polyharnstoffurethans wie in Anspruch 1 definiert, umfassend:

(a) Umsetzen von

(i) einem schwefelhaltigen Polycyanat und einem aktiven Wasserstoff enthaltenden Material oder
(ii) einem Polyisocyanat mit einem schwefelhaltigen aktiven Wasserstoff enthaltenden Material,

um ein Polyurethanpräpolymer zu bilden und
(b) Umsetzen dieses Polyurethanpräpolymers mit einem aminhaltigen Härtungsmittel,

wobei dieses aminhaltige Härtungsmittel eine Mischung aus einem Polyamin und mindestens einem Material ausgewählt aus einem Polythiol und Polyol ist.

**22.** Verfahren nach Anspruch 21, wobei das schwefelhaltige Polycyanat, das Polyisocyanat, das aktiven Wasserstoff enthaltende Material, das schwefelhaltige aktiven Wasserstoff enthaltende Material und das aminhaltige Härtungsmittel wie in einem der Ansprüche 2 bis 20 definiert sind.

**23.** Optischer Gegenstand, der ein schwefelhaltiges Polyharnstoffurethan wie in einem der Ansprüche 1 bis 20 definiert enthält.

**24.** Optischer Gegenstand nach Anspruch 23, der aus einer ophtalmischen Linse und einem photochromen Gegenstand ausgewählt ist.

**25.** Optischer Gegenstand nach Anspruch 24, der ein photochromer Gegenstand ist, wobei er mindestens ein teilweise gehärtetes Substrat und mindestens eine photochrome Menge einer photochromen Substanz aufweist.

**26.** Photochromer Gegenstand nach Anspruch 25, wobei diese photochrome Substanz mindestens teilweise in dieses Substrat imbibiert ist.

**27.** Photochromer Gegenstand nach Anspruch 25, wobei dieses Substrat mindestens teilweise mit einer Beschichtungszusammensetzung beschichtet ist, die mindestens eine photochrome Menge einer photochromen Substanz enthält.

**28.** Photochromer Gegenstand nach Anspruch 25, wobei diese photochrome Substanz aus Spiro(indolin)naphthoxazinen, Spiro(indolin)benzoxazinen, Benzopyranen, Naphthopyranen, Organometalldithizonaten, Fulgiden und Fulgimiden und Mischungen derselben ausgewählt ist.

## Revendications

**1.** Poly(urée-uréthane) soufré, adapté pour présenter, à l'état au moins partiellement durci, un indice de réfraction, déterminé selon la norme ASTM D 542-00, d'au moins 1,57, un nombre d'Abbe, déterminé selon la norme ASTM D 542-00, d'au moins 32, et une masse volumique inférieure à 1,3 g/$cm^3$, qu'on a préparé en faisant réagir :

a) un prépolymère polyuréthane soufré, qui est le produit de réaction

aa1) d'un polycyanate soufré
aa2) et d'une matière à hydrogène actif,
ou bien
ab1) d'un polyisocyanate
ab2) et d'une matière soufrée à hydrogène actif,

b) et un agent durcisseur aminé,

étant entendu que ledit agent durcisseur aminé est un mélange d'une polyamine et d'au moins un corps choisi parmi un polythiol (pb) et un polyol, et que

i) le polyuréthane soufré est le produit de réaction d'un polyisocyanate (ab1) et d'une matière soufrée à hydrogène actif (ab2) qui est un polythiol (pa) comprenant au moins un composé représenté par l'une des formules structurales suivantes :

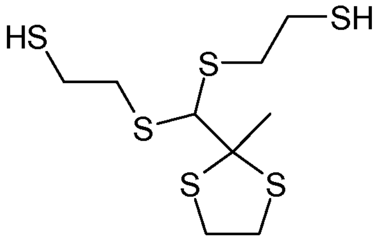
(IV'a)

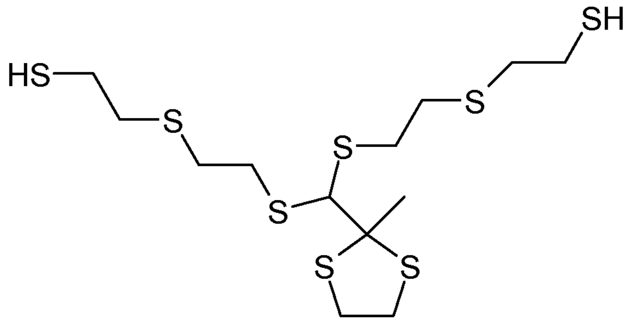
(IV'b)

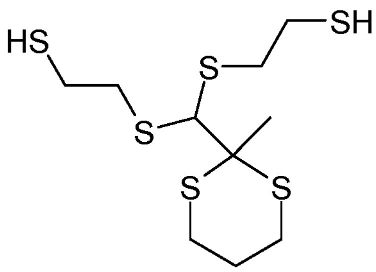
(IV'c)

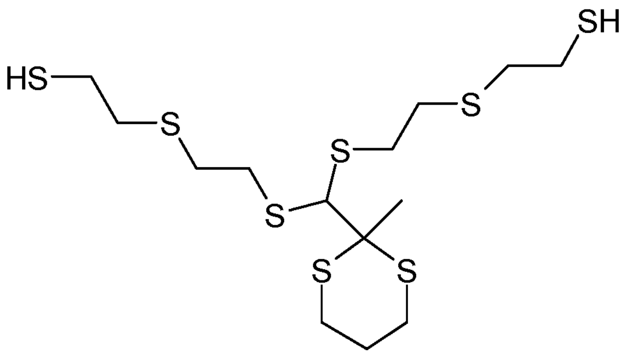
(IV'd)

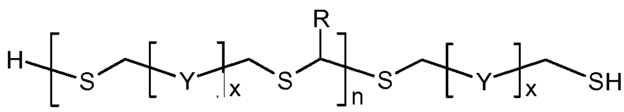
(IV'e)

dans laquelle

- R peut représenter un groupe méthyle, acétyle, alkyle en $C_1$-$C_{10}$, cycloalkyle, aryl-alkyle, ou alkyl-carbonyle,
- Y peut représenter un groupe alkyle en $C_1$-$C_{10}$, cycloalkyle ou aryle en $C_6$-$C_{14}$, ou de formule $(CH_2)_p(S)_m(CH_2)_q$, $(CH_2)_p(Se)_m(CH_2)_q$ ou $(CH_2)_p(Te)_m(CH_2)_q$, où l'indice m peut être un nombre entier valant de 1 à 5 et les indices p et q peuvent chacun être un nombre entier valant de 1 à 10,
- l'indice n peut être un nombre entier valant de 1 à 30,
- et l'indice x peut être un nombre entier valant de 0 à 10,

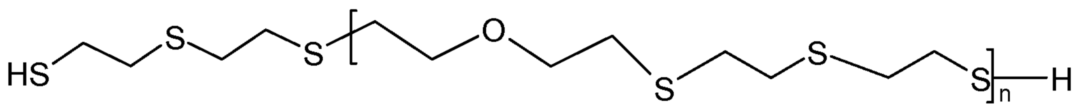
(IV'f)

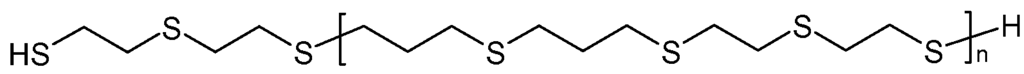

(IV'g)

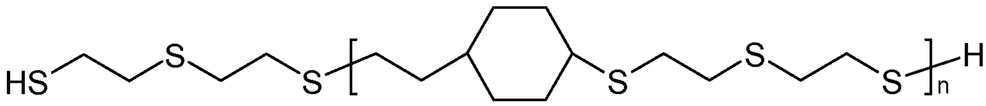

(IV'h)

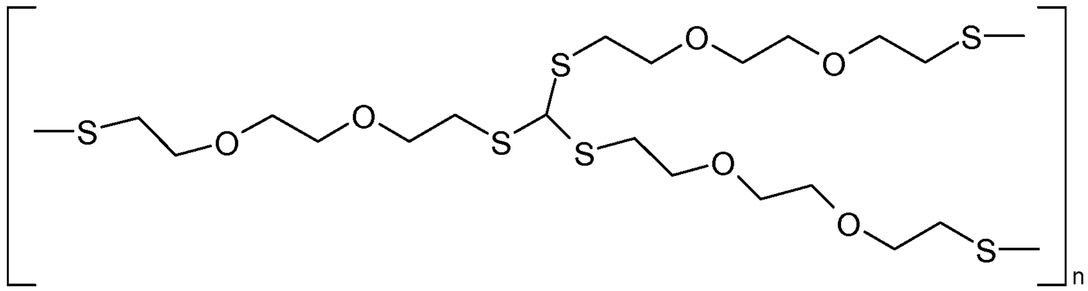

(IV'i)

dans lesquelles l'indice n peut être un nombre entier valant de 1 à 20,

ii) et/ou que le polythiol (pb) comprend au moins un composé représenté par l'une des formules structurales (IV'a) à (IV'i) données ci-dessus.

**2.** Poly(urée-uréthane) soufré conforme à la revendication 1, pour lequel ledit polycyanate soufré comprend un polyisothiocyanate ou un mélange d'un polyisothiocyanate et d'un polyisocyanate.

**3.** Poly(urée-uréthane) soufré conforme à la revendication 1, pour lequel la matière à hydrogène actif comprend :

a) un polyol non soufré,
b) un polythiol,
c) un mélange d'un polyol non soufré et d'un polythiol,
d) ou un polysulfure à groupe(s) fonctionnel(s) hydroxyle.

**4.** Poly(urée-uréthane) soufré conforme à la revendication 3, pour lequel ledit polysulfure à groupe(s) fonctionnel(s) hydroxyle comporte en outre un ou des groupe(s) fonctionnel(s) sulfhydryle SH.

**5.** Poly(urée-uréthane) soufré conforme à la revendication 3, pour lequel ledit polyol non soufré est choisi parmi les polyesterpolyols, polycaprolactone-polyols, polyéther-polyols, polycarbonate-polyols et leurs mélanges, et de préférence, ce polyol non soufré comprend un polyéther-polyol.

**6.** Poly(urée-uréthane) soufré conforme à la revendication 1, pour lequel ladite matière à hydrogène actif présente une masse molaire moyenne en nombre, déterminée par GPC, de 200 à 32 000 g/mol, et de préférence de 2 000 à 15 000 g/mol.

**7.** Poly(urée-uréthane) soufré conforme à la revendication 3, pour lequel ledit polyol non soufré comprend un polyéther-polyol.

**8.** Poly(urée-uréthane) soufré conforme à la revendication 7, pour lequel ledit polyéther-polyol est représenté par la formule structurale suivante :

$$H-(O-CRR-CRR-Y_n)_a-(CRR-CRR-Y_n-O)_b-(CRR-CRR-Y_n-O)_c-H$$

dans laquelle

- R peut représenter un atome d'hydrogène ou un groupe alkyle en $C_1$-$C_6$,
- Y peut représenter un chaînon de formule $CH_2$,
- l'indice n peut être un nombre entier valant de 0 à 6,
- et les indices a, b et c peuvent chacun être un nombre entier valant de 0 à 300, étant entendu que ces indices a, b et c sont choisis tels que la masse molaire moyenne en nombre du polyol, déterminée par GPC, n'excède pas 32 000 g/mol.

**9.** Poly(urée-uréthane) soufré conforme à la revendication 1, pour lequel ledit polycyanate soufré et ladite matière à hydrogène actif sont présents en des quantités telles que le rapport molaire des équivalents (NCO + NCS) aux équivalents (SH + OH) ou (SH + OH + NR) où R représente un atome d'hydrogène ou un groupe alkyle, est inférieur à 5,5/1,0.

**10.** Poly(urée-uréthane) soufré conforme à la revendication 1, pour lequel le polyisocyanate est choisi parmi les polyisocyanates aliphatiques, les polyisocyanates cycloaliphatiques, les polyisocyanates aromatiques, et les mélanges de tels composés.

**11.** Poly(urée-uréthane) soufré conforme à la revendication 1, pour lequel ledit polyisocyanate est choisi parmi les diisocyanates aliphatiques, les diisocyanates cycloaliphatiques, les diisocyanates aromatiques, leurs dimères cycliques et leurs trimères cycliques, ainsi que les mélanges de tels composés.

**12.** Poly(urée-uréthane) soufré conforme à la revendication 1, pour lequel ledit polyisocyanate est choisi parmi les suivants : cyclo-hexyl-méthane-diisocyanate et mélanges d'isomères de ce composé, isomère trans,trans du 4,4'-méthylène-bis(cyclohexyl-isocyanate), 3-(isocyanato-méthyl)-3,5,5-triméthyl-cyclohexyl-isocyanate, méta-tétraméthyl-xylène-diisocyanate (1,3-bis(1-isocyanato-1-méthyl-éthyl)-benzène), et mélanges de 3-(isocyanato-méthyl)-3,5,5-triméthyl-cyclo-hexyl-isocyanate et de méta-tétraméthyl-xylène-diisocyanate (1,3-bis-(1-isocyanato-1-méthyl-éthyl)-benzène).

**13.** Poly(urée-uréthane) soufré conforme à la revendication 1, pour lequel la matière soufrée à hydrogène actif est une matière qui porte un ou des groupe(s) sulfhydryle (SH), et de préférence un polythiol.

**14.** Poly(urée-uréthane) soufré conforme à la revendication 1 ou 13, pour lequel ledit polythiol est choisi parmi les polythiols aliphatiques, les polythiols cycloaliphatiques, les polythiols aromatiques, les polythiols polymères, les polythiols comportant des raccords de type éther, et les polythiols comportant un ou plusieurs raccord(s) de type sulfure.

**15.** Poly(urée-uréthane) soufré conforme à la revendication 13, pour lequel la matière qui porte un ou des groupe(s) sulfhydryle (SH) comprend un mélange de polythiol et de polyol non soufré.

**16.** Poly(urée-uréthane) soufré conforme à la revendication 1, pour lequel la matière soufrée à hydrogène actif est un polysulfure à groupe(s) fonctionnel(s) hydroxyle, lequel polysulfure à groupe(s) fonctionnel(s) hydroxyle, de préférence, porte en outre un ou des groupe(s) fonctionnel(s) sulfhydryle (SH).

**17.** Poly(urée-uréthane) soufré conforme à la revendication 1, pour lequel ladite polyamine comporte au moins deux groupes fonctionnels choisis, indépendamment, parmi les groupes amine primaire ($-NH_2$), amine secondaire (-NH-), et leurs combinaisons.

**18.** Poly(urée-uréthane) soufré conforme à la revendication 17, pour lequel ladite polyamine est choisie parmi les polyamines aliphatiques, les polyamines cycloaliphatiques, les polyamines aromatiques et les mélanges de telles polyamines.

**19.** Poly(urée-uréthane) soufré conforme à la revendication 17, pour lequel ladite polyamine est représentée par la formule structurale suivante, ou est un mélange de telles polyamines :

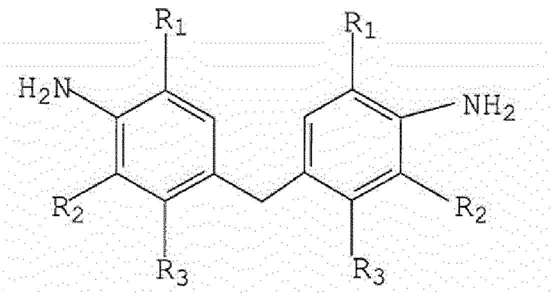

dans laquelle

- $R_1$ et $R_2$ représentent chacun, indépendamment, une entité choisie parmi les groupes méthyle, éthyle, propyle et isopropyle,
- et $R_3$ représente une entité choisie parmi un atome d'hydrogène et un atome de chlore.

**20.** Poly(urée-uréthane) soufré conforme à la revendication 17, pour lequel ladite polyamine est choisie parmi les suivantes : 4,4'-méthylène-bis(3-chloro-2,3-diéthyl-aniline), 2,4-diamino-3,5-diéthyl-toluène, 2,6-diamino-3,5-diéthyl-toluène, et mélanges de 2,4-diamino-3,5-diéthyl-toluène et de 2,6-diamino-3,5-diéthyl-toluène.

**21.** Procédé de préparation d'un poly(urée-uréthane) soufré conforme à la revendication 1, qui comporte les étapes suivantes :

a) faire réagir

i) un polycyanate soufré et une matière à hydrogène actif,
ii) ou un polyisocyanate et une matière soufrée à hydrogène actif, pour préparer un prépolymère polyuréthane,

b) et faire réagir ce prépolymère polyuréthane avec un agent durcisseur aminé,

étant entendu que ledit agent durcisseur aminé est un mélange d'une polyamine et d'au moins un corps choisi parmi un polythiol et un polyol.

**22.** Procédé conforme à la revendication 21, dans lequel le polycyanate soufré, le polyisocyanate, la matière à hydrogène actif, la matière soufrée à hydrogène actif et l'agent durcisseur aminé sont tels que définis dans l'une des revendications 2 à 20.

**23.** Article optique, comprenant un poly(urée-uréthane) soufré conforme à l'une des revendications 1 à 20.

**24.** Article optique conforme à la revendication 23, choisi parmi une lentille ophtalmique et un article photochrome.

**25.** Article optique conforme à la revendication 24, qui est un article photochrome qui comporte un substrat au moins partiellement durci et une substance photochrome, au moins en une quantité à effet photochrome.

**26.** Article photochrome conforme à la revendication 25, dans lequel au moins une partie de ladite substance photochrome a pénétré dans ledit substrat, par imbibition.

**27.** Article photochrome conforme à la revendication 25, dans lequel ledit substrat est revêtu, au moins en partie, d'une composition de revêtement comprenant une substance photochrome, au moins en une quantité à effet photochrome.

**28.** Article optique conforme à la revendication 25, dans lequel ladite substance photochrome est choisie parmi les suivantes : spiro-(indoline)naphtoxazines, spiro(indoline)benzoxazines, benzopyranes, naphtopyranes, dithizonates organo-métalliques, fulgides et fulgimides, ainsi que leurs mélanges.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2644007 A
- US 2680127 A
- US 6187444 B1
- US 4160853 A
- US 6509418 B1
- WO 03042270 A
- WO 0166623 A1
- US 5225472 A
- US 5693738 A
- US 3562172 A
- US 3578602 A
- US 4215010 A
- US 4342668 A
- US 5405958 A
- US 4637698 A
- US 4931219 A
- US 4816584 A
- US 4880667 A
- US 4818096 A
- US 3567605 A
- US 4826977 A
- US 5066818 A
- US 5466398 A
- US 5384077 A
- US 5238931 A
- US 5274132 A
- US 5429774 A
- US 3361706 A
- US 4931220 A
- US 4166043 A
- US 4367170 A


### Non-patent literature cited in the description


- **D.M. YOUNG ; F. HOSTETTLER et al.** Polyesters from Lactone. *Union Carbide,* vol. 40, 14
- *CHEMICAL ABSTRACTS,* 96-27-5
- Ullmann's Encyclopedia of Industrial Chemistry. 1992, vol. A21, 673-674